(19) Europäisches Patentamt
European Patent Office
Office européen des brevets

(11) EP 4 477 659 B1

(12) **EUROPEAN PATENT SPECIFICATION**

(45) Date of publication and mention of the grant of the patent:
**03.06.2026 Bulletin 2026/23**

(21) Application number: **23882170.6**

(22) Date of filing: **14.07.2023**

(51) International Patent Classification (IPC):
***C07H 1/00*** *(2006.01)* ***C07H 21/02*** *(2006.01)*
***C40B 50/14*** *(2006.01)*

(52) Cooperative Patent Classification (CPC):
**C07H 1/00; C07H 21/02; C07H 21/04;** Y02P 20/55

(86) International application number:
**PCT/JP2023/026108**

(87) International publication number:
**WO 2024/089953 (02.05.2024 Gazette 2024/18)**

(54) **METHOD FOR PRODUCING OLIGONUCLEOTIDE**

VERFAHREN ZUR HERSTELLUNG EINES OLIGONUKLEOTIDS

PROCÉDÉ DE PRODUCTION D’OLIGONUCLÉOTIDE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **27.10.2022 JP 2022172158**

(43) Date of publication of application:
**18.12.2024 Bulletin 2024/51**

(73) Proprietor: **Sumitomo Chemical Company, Limited**
**Tokyo 103-6020 (JP)**

(72) Inventor: **TANAKA, Yuki**
**Oita-shi, Oita 870-0106 (JP)**

(74) Representative: **Vossius & Partner Patentanwälte Rechtsanwälte mbB**
**Siebertstrasse 3**
**81675 München (DE)**

(56) References cited:
**WO-A1-2019/208571 WO-A1-2022/103842**
**WO-A1-2022/172994 WO-A2-2020/227618**
**JP-A- 2005 520 547 US-B1- 10 072 261**

- **ZHOU XUAN ET AL: "Development of Kilogram-Scale Convergent Liquid-Phase Synthesis of Oligonucleotides", vol. 87, no. 4, 22 November 2021 (2021-11-22), United States, pages 2087 - 2110, XP093269759, ISSN: 0022-3263, Retrieved from the Internet <URL:https://pubs.acs.org/doi/pdf/10.1021/acs.joc.1c01756> DOI: 10.1021/acs.joc.1c01756**
- **MITSUAKI SEKIGUCHI: "Consideration for Impurities Contained in Oligonucleotide Therapeutics", PHARMACEUTICAL AND MEDICAL DEVICE REGULATORY SCIENCE, vol. 51, no. 1, 1 January 2020 (2020-01-01), pages 11 - 21, XP093165672**
- **N. M. EL ZAHAR; N. MAGDY; A. M. EL-KOSASY; MICHAEL G. BARTLETT: "Chromatographic approaches for the characterization and quality control of therapeutic oligonucleotide impurities", BIOMEDICAL CHROMATOGRAPHY, JOHN WILEY & SONS LTD., GB, vol. 32, no. 1, 4 October 2017 (2017-10-04), GB , pages e4088, XP071550514, ISSN: 0269-3879, DOI: 10.1002/bmc.4088**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 477 659 B1

## Description

TECHNICAL FIELD

This application claims priority to and the benefit of Japanese Patent Application No. 2022-172158 filed October 27, 2022 according to the Paris Convention

The present invention relates to a production method for an oligonucleotide using the phosphoramidite method in solid-phase synthesis in which a thiol compound is used as a cation scavenger.

BACKGROUND ART

In recent years, an attention in the application of nucleic acid molecules to the medical field has been increased. Examples include antisense nucleic acids, aptamers, ribozymes, and nucleic acids which induce RNA interference (RNAi) such as siRNA, and the others, which are called nucleic acid therapeutics.

The synthesis of oligonucleotides can be produced using the phosphoramidite method (hereinafter, referred to as the "amidite method"), and the synthesis method comprises a step of deprotecting the protecting group for the hydroxy group at the 5' terminal. The step is conducted by reacting an oligonucleotide wherein the hydroxy group at the 5' terminal is protected with an acid. This reaction is an equilibrium reaction in which the protecting group for the hydroxy group at the 5' terminal is released as a cation, and is a reversible reaction.

In liquid-phase synthesis, the cation of the deprotected protecting group (for example, 4,4'-dimethoxytrityl cation) increases in the system with the progress of the deprotection reaction, which inhibits the progress of the equilibrium reaction of the leaving reaction. Accordingly, in order to proceed the reaction, a method using a cation scavenger is known (see Patent Literature 1).

On the other hand, in solid-phase synthesis, since such a cation is discharged to outside of the system without being retained in the system, it is known that the equilibrium reaction proceeds smoothly and the above-mentioned problems for liquid-phase synthesis may not occur (see Non-Patent Literature 1). Therefore, in solid-phase synthesis, there is no need for using the cation scavenger in general. In solid-phase synthesis, it has been reported a method of conducting the deprotection reaction using triethylsilane as a cation scavenger (see Patent Literature 2). However, the effect of triethylsilane as a cation scavenger in the method was not sufficient.

Patent Literature 3 describes a specific convergent liquid phase process for manufacturing oligonucleotides by coupling two or more oligonucleotide fragments, each of which have two or more nucleotides. Patent Literature 4 concerns specific reagents and processes for preparing oligonucleotides, which have two or more nucleotides.

Patent Literature 5 relates to a method for double coupling a nucleoside phosphoramidite during synthesis of an oligonucleotide, the method comprising:

(a) contacting a free hydroxyl group of a terminal nucleoside residue attached to a solid phase support with a first sample of a protected nucleoside phosphoramidite to couple the protected nucleoside to the terminal nucleoside residue via an internucleoside P(III) linkage;

(b) after step (a), exposing the contacted nucleoside residue to an oxidizing agent to oxidize the linkage and produce a first coupled and oxidized product;

(c) after step (b), contacting the first coupled and oxidized product with a second sample of the protected nucleoside phosphoramidite to couple the protected nucleoside to residual free hydroxyl groups of the terminal nucleoside residue via an internucleoside P(III) linkage; and

(d) after step (c), adding an oxidizing agent to oxidize the linkage and produce a protected terminal nucleoside residue.

Patent Literature 6 concerns an amidite compound represented by formula (1) which enables a synthesis of RNA, and a method for preparing a polynucleotide by using the same compound.

Patent Literature 7 relates to a method of separating a target oligonucleotide from an impurity, in a mixture comprising said target oligonucleotide and said impurity, using a titratable anion exchange composition, comprising the steps: a) binding said target oligonucleotide to said titratable anion exchange composition; b) passing a solution through said titratable anion exchange composition with target oligonucleotide bound thereon, wherein said solution increases in pH over time; and c) eluting said target oligonucleotide, wherein said impurity elutes at a different pH than said target oligonucleotide.

Patent Literature 8 concerns a method for producing an oligonucleotide having a phosphate ester bond by oxidizing an oligonucleotide precursor having a phosphite ester bond or a phosphonate ester bond with an oxidant, wherein the oxidant is a compound of a certain formula (I).

Non-Patent Literature 2 describes the development of kilogram-scale convergent liquid-phase synthesis of oligonucleotides. Non-Patent Literature 3 includes consideration for impurities contained in oligonucleotide therapeutics. Non-Patent Literature 4 concerns chromatographic approaches for the characterization and quality control of therapeutic oligonucleotide impurities.

CITATION LIST

PATENT LITERATURE

Patent Literature 1: WO 2012/157723 A1
Patent Literature 2: US 5,510,476 B1
Patent Literature 3: WO 2020/227618 A2
Patent Literature 4: WO 2022/103842 A1
Patent Literature 5: US 10 072 261 B1
Patent Literature 6: WO 2019/208571 A1
Patent Literature 7: JP 2005 520547 A
Patent Literature 8: WO 2022/172994 A1

NON-PATENT LITERATURE

Non-Patent Literature 1: J. Am. Chem. Society., 2020, 142, 16610

Non-Patent Literature 2: Zhou Xuan et al., The Journal of Organic Chemistry, 2021, 87(4), pages 2087-2110.

Non-Patent Literature 3: Mitsuaki Sekiguchi, Pharmaceutical and Medical Device Regulatory Science, 2020, 51(1), pages 11 -21.

Non-Patent Literature 3: N. M. El Zahar; et al., Biomedical Chromatography, 2017, 32(1), page e4088.

SUMMARY OF THE INVENTION

(PROBLEMS TO BE SOLVED BY INVENTION)

The present invention aims to provide a production method for an oligonucleotide using the phosphoramidite method in solid-phase synthesis, in which the deprotection reaction of the hydroxy group at the 5' terminal proceeds efficiently and the yield and purity of the synthesized oligonucleotide are improved. The present invention also aims to provide an oligonucleotide in which the content ratio of N-1 mer in the oligonucleotide is significantly low.

(MEANS TO SOLVE PROBLEMS)

The present inventors, as a result of intensive studies to achieve the above objective, found out that by using a thiol compound as a cation scavenger, the deprotection reaction of the protecting group for the 5' hydroxy group proceeds efficiently and the yield and purity of the obtained oligonucleotide are improved. As a result, the present invention provides a production method for an oligonucleotide by solid-phase synthesis method, which comprises a step of reacting an oligonucleotide wherein the hydroxy group at the 5' terminal is protected with a protecting group that can be removed under acidic conditions characterised in that the reaction is performed with an acid in the presence of a thiol to remove the hydroxy group at the 5' terminal.

The present invention encompasses the following aspects, but which are not limited thereto.

[1] A production method for an oligonucleotide by solid-phase synthesis method, which comprises reacting an oligonucleotide wherein the hydroxy group at the 5' terminal is protected with a protecting group that can be removed under acidic conditions characterised in that the reaction is performed with an acid in the presence of a thiol to remove the protecting group for the hydroxy group at the 5' terminal (hereinafter, referred to as "Production method of the present invention").
[2] The production method according to [1] wherein the thiol is a C2-C20 alkyl thiol, or a C4-C8 cycloalkyl thiol.
[3] The production method according to [1] or [2] wherein the thiol is 1-dodecanethiol or cyclohexanethiol.
[4] The production method according to any one of [1] to [3] wherein the protecting group for the hydroxy group at the 5'

terminal is a protecting group represented by the following formula:

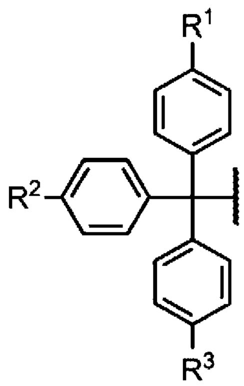

[wherein $R^1$, $R^2$ and $R^3$, each independently identical to or different from each other, represent a hydrogen atom or an alkoxy group).

[5] The production method according to any one of [1] to [4] wherein the protecting group for the hydroxy group at the 5' terminal is a 4,4'-dimethoxytrityl group (DMTr group).

[6] The production method according to any one of [1] to [5] wherein the acid is trifluoroacetic acid, dichloroacetic acid, trifluoromethanesulfonic acid, trichloroacetic acid, methanesulfonic acid, hydrochloric acid, acetic acid, or p-toluenesulfonic acid.

[7] The production method according to any one of [1] to [5] wherein the acid is dichloroacetic acid.

[8] The production method according to [7] wherein the acid is dichloroacetic acid in the co-existence of aprotic inert solvent having a lower boiling point than dichloroacetic acid.

[9] The production method according to [8] wherein the aprotic inert solvent having a lower boiling point than dichloroacetic acid is one or more solvents selected from dichloromethane, acetonitrile, or an aromatic organic solvent.

[10] The production method according to [9] wherein the aromatic organic solvent is toluene.

[11] The production method according to [7] wherein the dichloroacetic acid is dichloroacetic acid wherein the molar ratio of formaldehyde to dichloroacetic acid is $81 \times 10^{-5}$ or less, and the molar ratio of dichloroacetic anhydride to dichloroacetic acid is $20 \times 10^{-5}$ or less.

[12] The production method according to any one of [1] to [11], wherein the oligonucleotide wherein the hydroxy group at the 5' terminal is protected with the protecting group that can be removed under acidic conditions is an oligonucleotide represented by formula (1):

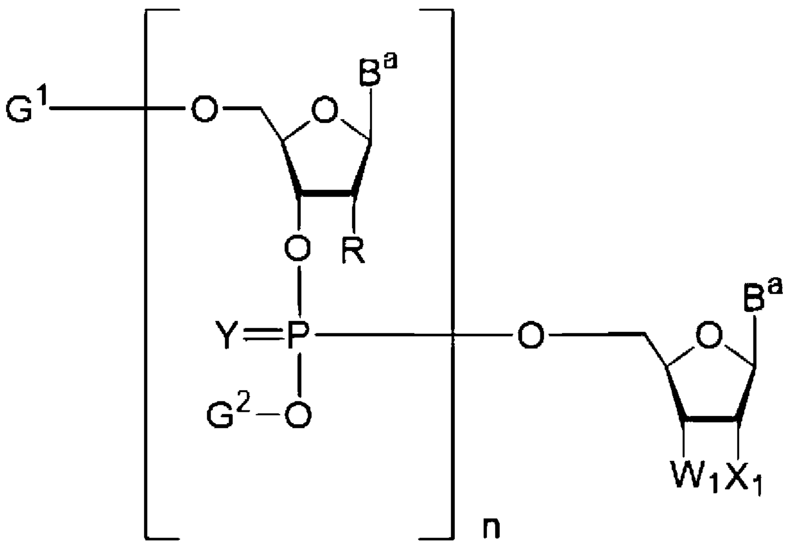

(1)

(wherein

$G^1$ represents a protecting group for a hydroxy group,

$G^2$ represents a protecting group for a hydroxy group,
$B^a$ represents a nucleobase, each independently identical to or different from each other, which may be protected with a protecting group,
R, each independently identical to or different from each other, represents a protected hydroxy group, a hydrogen atom, a fluorine atom, a methoxy group, a 2-methoxyethyl group, or an OQ' group,
Q', each independently identical to or different from each other, represents a methylene group which is bonded to the carbon atom at the 4' position of the ribose, an ethylene group which is bonded to the carbon atom at the 4' position of the ribose, or an ethylidene group which is bonded to the carbon atom at the 4' position of the ribose,
Y, each independently identical to or different from each other, represents an oxygen atom or a sulfur atom,
n represents any integer of 1 to 300,
$W_1$ represents an OZ group and $X_1$ represents an R group, or
$W_1$ represents an OV group and $X_1$ represents an OZ group,
V represents a protecting group for a hydroxy group,
Z is a group having a structure consisting of a solid support and a connecting group, and
when n is an integer of 2 or more, a non-nucleotide linker may be incorporated between respective nucleotides in the nucleic acid molecule represented by formula (1)), and
the nucleotide wherein the protecting group for the hydroxy group at the 5' terminal is removed is an oligonucleotide represented by formula (2):

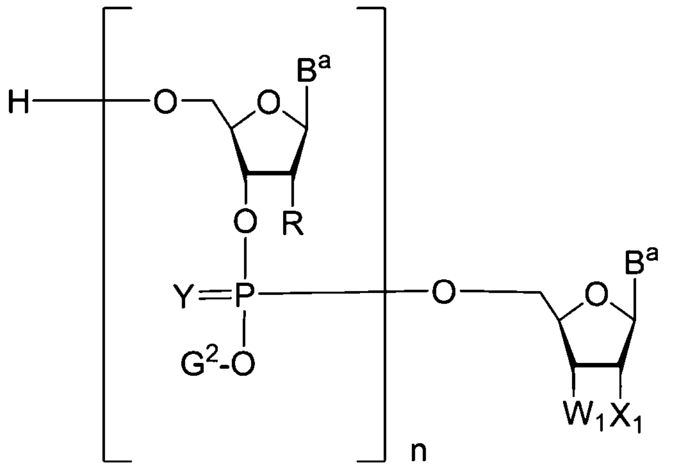

(2)

(wherein

$G^2$, $B^a$, R, Y, $X_1$, $W_1$ and n are as described above, and
as defined in formula (1), a non-nucleotide linker may be incorporated between nucleotides).

[13] The production method according to [12], wherein the oligonucleotide wherein the hydroxy group at the 5' terminal is protected with the protecting group that can be removed under acidic conditions is an oligonucleotide represented by formula (1'):

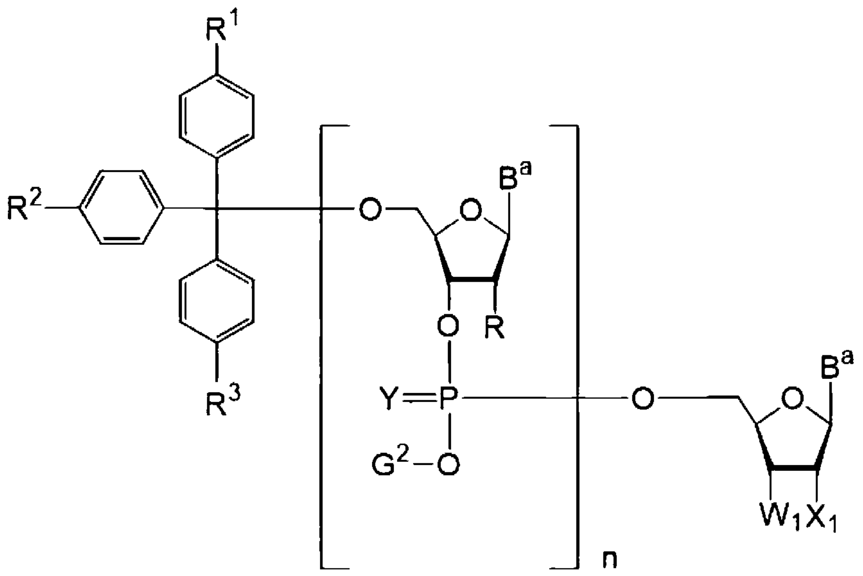

(1')

(wherein

$G^2$, $B^a$, R, Y, $X_1$, $W_1$ and n are as described above, and
$R^1$, $R^2$ and $R^3$, each independently identical to or different from each other, represent a hydrogen atom or an alkoxy group) .

[14] The production method according to [13] wherein $R^1$ and $R^2$ are methoxy groups, and $R^3$ is a hydrogen atom.
[15] A production method for producing an oligonucleotide represented by formula (2'):

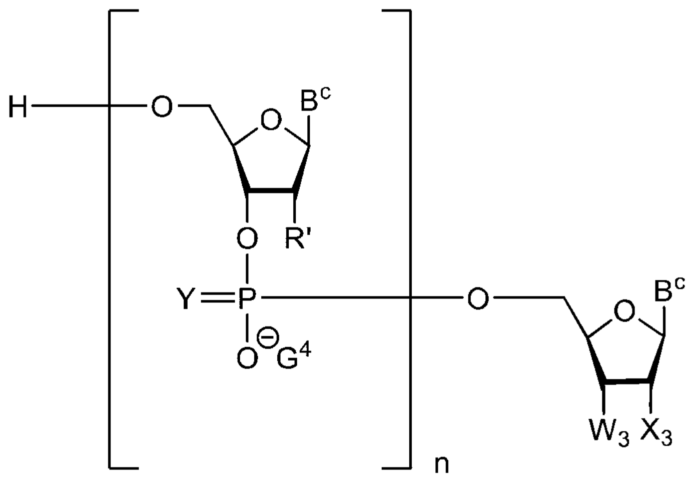

( 2' )

(wherein

Y and n are as described above,
$B^c$, each independently identical to or different from each other, represents a nucleobase,
$G^4$, each independently identical to or different from each other, represents a hydrogen ion, an alkali metal ion, an ammonium ion, an alkyl ammonium ion, or a hydroxy alkyl ammonium ion,
R', each independently identical to or different from each other, represents a hydroxy group, a hydrogen atom, a fluorine atom, a methoxy group, a 2-methoxyethyl group, or an OQ' group,
Q' is as described above, and
$X_3$ and $W_3$ each independently represent a hydroxy group, or
$X_3$ represents an R' group and $W_3$ represents a hydroxy group, and
as defined in formula (1), a non-nucleotide linker may be incorporated between nucleotides)

which comprises the step described in [12] and further a step of removing a group represented by Z from the oligonucleotide represented by formula (2) which is produced by the step, and a step of removing protecting groups for hydroxy groups and nucleobases.
[16] The production method according to any one of [12] to [15] wherein the oligonucleotide is an oligonucleotide containing ribonucleoic acid (RNA).
[17] The production method according to [12] wherein the oligonucleotide is an oligonucleotide containing ribonucleic acid (RNA), and a protecting group for the hydroxy group at the 2' position of the ribose is a protecting group represented by formula (6):

formula (6):

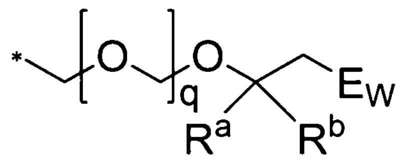

(6)

(wherein

q represents any integer of 0 to 5,
$R^a$ and $R^b$, each independently identical to or different from each other, represent a methyl group, an ethyl group or a hydrogen atom,
mark * represents the bonding point with the oxygen atom derived from the hydroxy group at the 2' position of the ribose, and
$E_W$ represents an electron-withdrawing group).

[18] The production method according to [17] wherein q is 0 or 1, $R^a$ and $R^b$, each independently identical to or different from each other, represent a methyl group or a hydrogen atom, and $E_W$ is a cyano group.
[19] The production method according to any one of [12] to [18] wherein n is any integer of 1 to 200.
[20] The production method according to any one of [1] to [19] wherein the obtained oligonucleotide is an oligonucleotide of 100 mer or more.
[20-1] The production method according to any one of [1] to [19] wherein the obtained oligonucleotide is an oligonucleotide of 20 mer or more.
[20-2] The production method according to any one of [1] to [19] wherein the obtained oligonucleotide is an oligonucleotide of 40 mer or more.
[20-3] The production method according to any one of [1] to [19] wherein the obtained oligonucleotide is an oligonucleotide of 60 mer or more.
[20-4] The production method according to any one of [1] to [19] wherein the obtained oligonucleotide is an oligonucleotide of 80 mer or more.
[20-5] The production method according to any one of [1] to [19] wherein the obtained oligonucleotide is an oligonucleotide of 150 mer or more.
[20-6] The production method according to any one of [1] to [19] wherein the obtained oligonucleotide is an oligonucleotide of 200 mer or more.
[20-7] The production method according to any one of [1] to [19] wherein the obtained oligonucleotide is an oligonucleotide of 250 mer or more.
[20-8] The production method according to any one of [1] to [19] wherein the obtained oligonucleotide is an oligonucleotide of 300 mer or more.
[21] The production method according to any one of [1] to [20] wherein the molar ratio of the used amounts of the thiol to the oligonucleotide wherein the hydroxy group at the 5' terminal is protected with the protecting group that can be removed under acidic conditions is 1 or more.
[22] The production method according to any one of [1] to [21] wherein the molar ratio of the used amounts of the thiol to the acid is 1 to 100.

(EFFECT OF INVENTION)

The present invention provides a production method for an oligonucleotide which comprises a step of effectively proceeding a deprotection reaction of a protecting group for a 5' hydroxy group by using a thiol compound as a cation

scavenger. According to the production method of the present invention, an improvement in the yield and purity of an oligonucleotide produced can be expected.

BRIEF DESCRIPTION OF DRAWINGS

[Fig. 1] Fig. 1 is Scheme A showing a typical example of producing a nucleic acid molecule represented by formula (5) from a nucleic acid molecule represented by formula (1). In the figure, $G^1$ may be used any groups without any particular limitations as long as it can function as a protecting group for a hydroxy group which is removed with dichloroacetic acid, and known protecting groups which are used for amidite compounds can be widely used. In addition, $G^3$, each independently identical to or different from each other, represents an alkyl group, or two $G^3$ may be bonded each other to form a cyclic structure. $G^3$, each independently identical to or different from each other, represents an alkyl group, preferably, for example, a methyl group, an ethyl group, a propyl group, or an isopropyl group, and more preferably, both of them represent an isopropyl group. The other symbols are as described above.

MODE FOR CARRYING OUT THE INVENTION

According to one embodiment of the present invention, the present invention relates to a production method for an oligonucleotide by solid-phase synthesis method, which comprises
a step of reacting an oligonucleotide wherein the hydroxy group at the 5' terminal is protected with a protecting group that can be removed under acidic conditions characterised in that the reaction is performed with an acid in the presence of a thiol to remove the protecting group for the hydroxy group at the 5' terminal.
As used herein, the term of "oligonucleotide wherein the hydroxy group at the 5' terminal is protected with a protecting group that can be removed under acidic conditions" refers to an oligonucleotide which comprises a nucleotide wherein the hydroxy group at the 5' terminal in the structure of a nucleotide molecule is protected with a protecting group that can be removed under acidic conditions. As used herein, "oligonucleotide" sometimes refers to "nucleic acid oligomer", and "nucleotide" sometimes refers to "nucleic acid molecule".
The term of "hydroxy group at the 5' terminal is a protecting group that can be removed under acidic conditions" may be any generally known protecting groups, which is not particularly limited thereto. Specific examples of the protecting group include a group represented by the following formula:

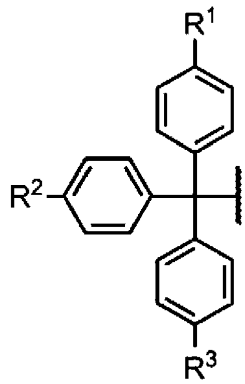

(wherein
$R^1$, $R^2$ and $R^3$, each independently identical to or different from each other, represent a hydrogen atom or an alkoxy group), however, which are not particularly limited thereto as long as they can be released as a cation.
In the protecting group represented by the above formula, a combination of $R^1$, $R^2$ and $R^3$ is preferably the combination wherein one of them is a hydrogen atom and the remaining two thereof are alkoxy groups which is identical to or different from each other (preferably identical), and a particularly preferable example of the alkoxy group includes a methoxy group.
Specific preferable examples of the protecting group include a group selected from a 4,4'-dimethoxytrityl group (DMTr group), a 4-monomethoxytrityl group, or a 4,4',4"-trimethoxytrityl group. The 4,4'-dimethoxytrityl group (DMTr group) is particularly preferable.
As used herein, specific examples of the term of "thiol" include a C2-C20 alkyl thiol, or a C4-C8 cycloalkyl thiol. Examples of the C2-C20 alkyl thiol include ethanethiol, 1-propanethiol, 2-propanethiol, 1-butanethiol, 2-butanethiol, 2-methyl-1-propanethiol (isobutylmercaptan), 2- methyl-2-propanethiol (tert-butylmercaptan), 1-pentanethiol, 1-hexanethiol, 1-heptanethiol, 1-octanethiol, 1-nonanethiol, 1-decanethiol, 1-undecanethiol, 1-dodecanethiol, tert-dodecanethiol, 1-tetradecanethiol, 1-pentadecanethiol, 1-hexadecanethiol, 1-octadecanethiol, and 1-eicosanethiol. Examples

of the C4-C8 cycloalkyl thiol include cyclobutanethiol, cyclopentanethiol, cyclohexanethiol, cycloheptanethiol, and cyclooctanethiol. Preferable examples of the thiol includes 1-dodecanethiol and cyclohexanethiol.

The molar ratio of used amounts of the thiol to the oligonucleotide wherein the hydroxy group at the 5' terminal is protected with the protecting group that can be removed under acidic conditions is 1 or more, but which is not limited thereto. In addition, the molar ratio of the thiol to the acid is 1 to 100, but which is not limited thereto.

The term "acid" means the acid which is used to remove the protecting groups in the phosphoramidite method, and specific examples thereof include trifluoroacetic acid, dichloroacetic acid, trifluoromethanesulfonic acid, trichloroacetic acid, methanesulfonic acid, hydrochloric acid, acetic acid, and p-toluenesulfonic acid, but which are not limited thereto. Dichloroacetic acid is particularly preferable.

It is preferable that the dichloroacetic acid used is that of high purity. A high purity dichloroacetic acid means that wherein the content of impurities contained in dichloroacetic acid is below a certain amount. Impurities include both or one of formaldehyde and dichloroacetic anhydride, as used in the production method for dichloroacetic acid.

For example, the molar ratio of formaldehyde to dichloroacetic acid is $81 \times 10^{-5}$ or less, preferably $41 \times 10^{-5}$ or less, and more preferably $81 \times 10^{-6}$ or less. Further, the molar ratio of dichloroacetic anhydride to dichloroacetic acid is $20 \times 10^{-5}$ or less, preferably $10 \times 10^{-5}$ or less, and more preferably $50 \times 10^{-6}$ or less.

According to an embodiment of the dichloroacetic acid used, it is preferable to use dichloroacetic acid, in which the molar ratio of formaldehyde to dichloroacetic acid is equal to or less than the above ratio, and/or the molar ratio of dichloroacetic anhydride to dichloroacetic acid is equal to or less than the above ratio.

The dichloroacetic acid may be in a liquid form (for example, solution, suspension, or emulsion) contained in an aprotic inert solvent having a lower boiling point than dichloroacetic acid, which is used in the process for producing and purifying dichloroacetic acid, or it may be used in the coexistence of an inert solvent by separately adding such an inert solvent to the reaction system.

Examples of the aprotic inert solvent having a boiling point lower than dichloroacetic acid include aprotic inert solvents having a boiling point of 181°C or lower, and specific examples thereof include dichloromethane, acetonitrile, and an aromatic organic solvent, but which are not limited thereto. Examples of the aromatic organic solvent include toluene, xylene, monochlorobenzene, and o-dichlorobenzene, include preferably toluene. The used amounts of such a solvent is not particularly limited, but a typical example thereof includes about 0.5 times by weight to 20 times by weight to the weight of dichloroacetic acid.

In addition, as necessary, in the reaction system containing dichloroacetic acid for the deprotection reaction, in the co-existence of an aprotic inert solvent having a boiling point lower than dichloroacetic acid, one compound selected from the group consisting of an aliphatic alcohol, an aliphatic amine, and water may be added.

Examples of the aliphatic alcohols and aliphatic amines having a boiling point lower than dichloroacetic acid include a C1-C6 aliphatic alcohol compound and a C1-C6 aliphatic amine compound. Examples of the C1-C6 aliphatic alcohol include methanol, ethanol, n-propanol, i-propanol, n-butanol, i-butanol, sec-butanol, t-butanol, n-pentanol, n-hexanol and the others. Examples of the aliphatic amine having a boiling point lower than dichloroacetic acid include the C1-C6 aliphatic amine compounds. Specific examples thereof include methylamine, ethylamine, n-propylamine, i-propylamine, n-butylamine, i-butylamine, t-butylamine, n-pentylamine, n-hexylamine and the others.

The used amounts of the aliphatic alcohol, the aliphatic amine, water, or a mixture thereof may be any amounts which are effective to reduce those of dichloroacetic anhydride to the desired range, but which is not particularly limited thereto.

Specific examples of the term of "oligonucleotide wherein the hydroxy group at the 5' terminal is protected with a protecting group that can be removed under acidic conditions" include an oligonucleotide represented by formula (1):

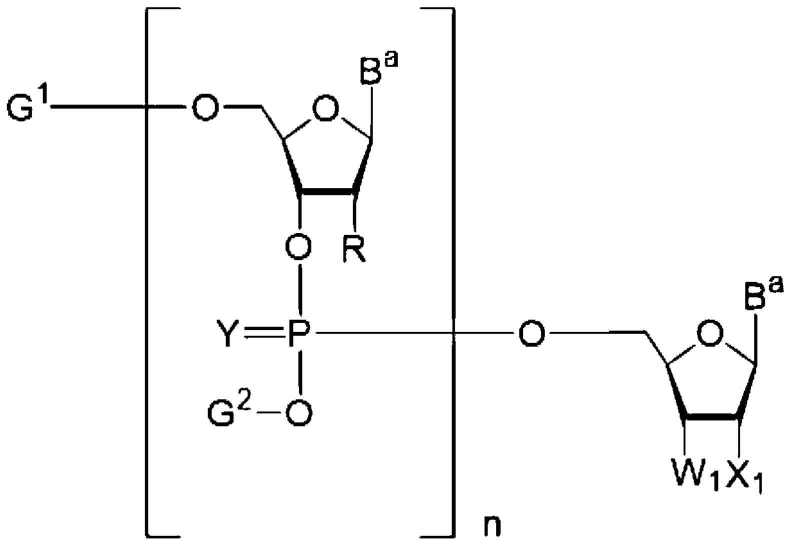

(1)

(wherein

$G^1$ represents a protecting group for a hydroxy group,
$G^2$ represents a protecting group for a hydroxy group,
$B^a$ represents a nucleobase, each independently identical to or different from each other, which may be protected with a protecting group,
R, each independently identical to or different from each other, represents a protected hydroxy group, a hydrogen atom, a fluorine atom, a methoxy group, a 2-methoxyethyl group, or an OQ' group,
Q', each independently identical to or different from each other, represents a methylene group which is bonded to the carbon atom at the 4' position of the ribose, an ethylene group which is bonded to the carbon atom at the 4' position of the ribose, or an ethylidene group which is bonded to the carbon atom at the 4' position of the ribose,
Y, each independently identical to or different from each other, represents an oxygen atom or a sulfur atom,
n represents any integer of 1 to 300,
$W_1$ represents an OZ group and $X_1$ represents an R group, or
$W_1$ represents an OV group and $X_1$ represents an OZ group,
V represents a protecting group for a hydroxy group,
Z is a group having a structure consisting of a solid support and a connecting group, and
when n is an integer of 2 or more, a non-nucleotide linker may be incorporated between respective nucleotides in the nucleic acid molecule represented by formula (1)).

According to more preferable embodiment of the present invention, an oligonucleotide wherein the hydroxy group at the 5' terminal is protected with a protecting group that can be removed under acidic conditions include an oligonucleotide represented by formula (1'):

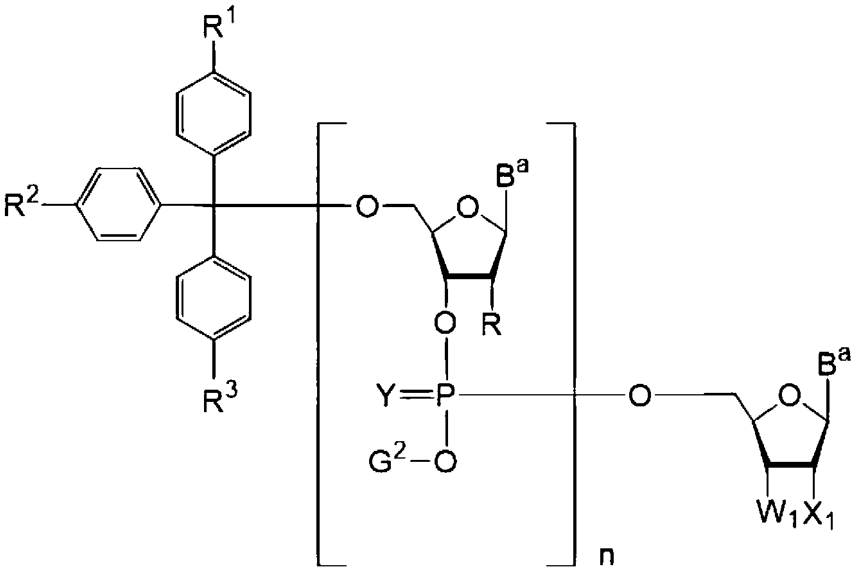

(1')

(wherein

$G^2$, $B^a$, R, Y, $X_1$, $W_1$ and n are as described above, and
$R^1$, $R^2$ and $R^3$, each independently identical to or different from each other, represent a hydrogen atom or an alkoxy group) .

According to further more preferable embodiment of the present invention, in formula (1'), $R^1$, $R^2$ and $R^3$ represent hydrogen atoms.

In addition, according to an embodiment of the present invention, a nucleotide wherein the protecting group for the hydroxy group at the 5' terminal is removed includes an oligonucleotide represented by formula (2):

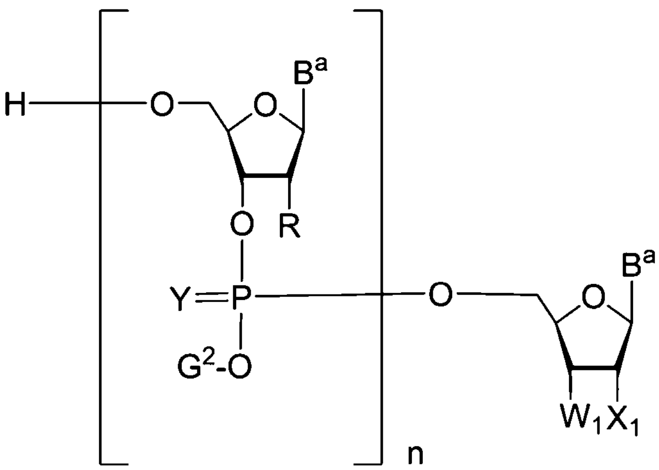

( 2 )

(wherein

$G^2$, $B^a$, R, Y, $X_1$, $W_1$ and n are as described above, and
as defined in formula (1), a non-nucleotide linker may be incorporated between nucleotides).

In addition, according to a preferable embodiment of the present invention, a nucleotide wherein the protecting group for the hydroxy group at the 5' terminal is removed includes an oligonucleotide represented by formula (2'):

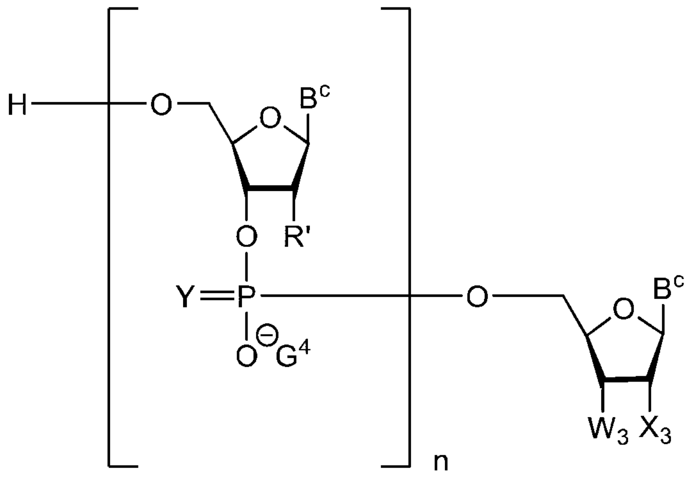

( 2' )

(wherein

Y and n are as described above,
$B^c$, each independently identical to or different from each other, represents a nucleobase,
$G^4$, each independently identical to or different from each other, represents a hydrogen ion, an alkali metal ion, an ammonium ion, an alkyl ammonium ion, or a hydroxy alkyl ammonium ion,
R', each independently identical to or different from each other, represents a hydroxy group, a hydrogen atom, a fluorine atom, a methoxy group, a 2-methoxyethyl group, or an OQ' group,
Q' is as described above, and
$X_3$ and $W_3$ each independently represent a hydroxy group, or
$X_3$ represents an R' group and $W_3$ represents a hydroxy group, and
as defined in formula (1), a non-nucleotide linker may be incorporated between nucleotides).

Examples of the protecting group for the hydroxy group at the 5' position of the nucleic acid molecule include the groups represented by $G^1$ or $G^5$ below. Examples of the oligonucleotide wherein the hydroxy group at the 5'-position is protected include the oligonucleotide represented by formula (1) (or including (1')). An example of the nucleotide produced by reacting the acid (for example, dichloroacetic acid) and a thiol include the oligonucleotide represented by formula (2) (or including (2')).

Specific examples of the compounds of formulas (1) and (2) wherein the group represented by Q' represents each independently identical to or different from each other and represents a methylene group bonded to the carbon atom at the 4' position of the ribose, an ethylene group bonded to the carbon atom at the 4' position of the ribose, or an ethylidene group bonded to the carbon atom at the 4' position of the ribose include the compounds of the structure represented by the following formula (7) as LNA-1, LNA-2 or LNA-3.

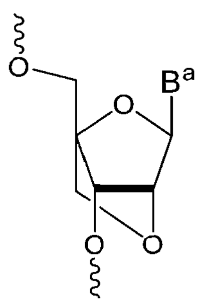

LNA-1

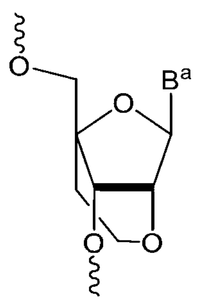

LNA-2

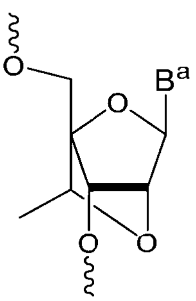

LNA-3

( 7 )

(wherein $B^a$ represents an optionally protected nucleobase).

More specific examples of the group represented by Z which has the structure consisting of the solid support and the connecting group connecting the solid support and the oxygen atom of the hydroxy group at the 2' position or 3' position of the ribose at the 3' terminal of the nucleic acid molecule include the group shown in the following formula (8).

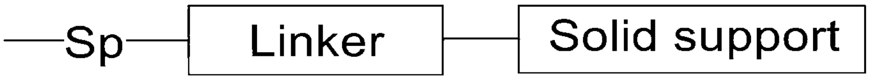

(8)

In formula (8), Sp represents a spacer.

Examples of the Spacer (Sp) include those having a structural formula shown in the following formula (9).

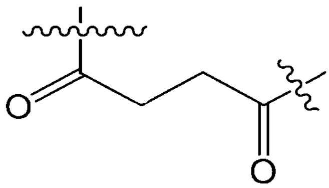
(9)

The Linker may be, for example, a structure shown in the following formula (10), or a structure in which the structure of formula (10) does not have a hexamethylene amino group moiety and an aminopropyl group is bonded to Si. Alternatively, the Linker may be a structure shown in the following formula (11).

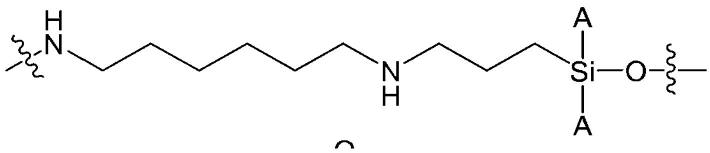
(10)

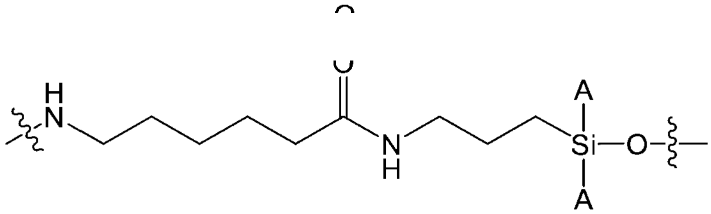
(11)

(wherein
A may be any of a hydroxy group, an alkoxy group, and an alkyl group. Examples of the alkoxy group include a methoxy group and an ethoxy group. Examples of the alkyl group include a methyl group, an ethyl group, an isopropyl group, and a n-propyl group. Si indicates that it is bonded to an oxygen atom of a hydroxy group on a support surface.)

Examples of the solid support include an inorganic porous support and an organic resin support. Example of the inorganic porous support includes Controlled Pore Glass (CPG). Example of the organic resin support includes a support made of polystyrene.

Example of the nucleoside (such as a ribose, and a deoxyribose) contained in the nucleic acid molecule used in the present invention includes DNA, RNA, 2'-O-MOE (2'-O-methoxyethyl), 2'-O-Me, 2'-F RNA, and the above LNA, but the nucleoside is not limited thereto.

The process for synthesizing the oligonucleotide (nucleic acid oligomer) by solid-phase synthesis method, which comprises the step of deprotecting the hydroxy group at the 5' terminal with the acid (for example, trichloroacetic acid) in the presence of the thiol, typically comprises the following steps.

(1) a step of deprotecting a hydroxy group at a 5' position of a nucleoside wherein a hydroxy group is protected and which is bonded to a solid support via a linker;
(2) a step of subjecting the hydroxy group at the 5' position produced in the previous step to a coupling reaction with a phosphoramidite compound to obtain a phosphite triester compound;
(3) a step of oxidizing the phosphite triester produced in the previous step to convert into a phosphate triester to produce an elongated nucleic acid oligomer, or an optional step of converting into a thiophosphate triester;
(4) a step of synthesizing a nucleic acid oligomer on the solid support by repeating, any number of times, a series of reaction cycles composed of the steps (1) to (3), that is, the step of deprotecting the hydroxy group at the 5' position of the produced nucleic acid oligomer, the step of coupling the hydroxy group at the 5' position with the amidite compound, and the step of oxidizing the produced phosphite triester; and
(5) a step of subjecting the nucleic acid oligomer on the solid support produced in the step (4) to a step of cleavage and deprotection to release it from the solid support to produce a nucleic acid oligomer with the protecting group removed therefrom. Here the process for synthesizing the nucleic acid oligomer may comprises subsequent to the step (2) or (3), a step of capping the hydroxy group at the 5' position where the coupling reaction with the phosphoramidite

compound did not proceed, or a capping step may be added between any steps in the series of reaction cycles composing of the step (4).

More specifically, in the step (5), the nucleic acid oligomer on the solid support produced in the step (4) is subjected to the reactions of the following steps (5-1) and (5-2) in order, and then subjected to the reaction of the step (5-3). Here, the reaction in the step (5-1) may be optionally conducted, and the reaction in the step (5-2) may be conducted according to the method described in JP 4705716 B. As a result, it is possible to produce a nucleic acid oligomer wherein protecting groups have been removed from the nucleic acid oligomer released from the solid support, or a nucleic acid oligomer wherein the hydroxy group at the 5' terminal is protected.

(5-1) a reaction of deprotecting the protecting group for the hydroxy group at the 5' terminal of a nucleic acid oligomer;
(5-2) a reaction of cleaving and releasing a nucleic acid oligomer from a solid support; and
(5-3) a reaction of deprotecting a protecting group for a hydroxy group at a 2' position or 3' position of a ribose composing of a nucleic acid oligomer.

The scheme of the steps (1) to (5) is shown in Fig. 1. The deprotection reaction in the step (1) or step (4) shown in Fig. 1 is conducted using the dichloroacetic acid and thiol compound. The definitions of the substituents in the chemical formulas in Scheme A are as defined above.

The nucleic acid oligomer of formula (1) may be further elongated by any chain length using a nucleotide type or non-nucleotide type of linker by the amidite method, and can be used to produce the nucleic acid oligomer represented by formula (3). It is also possible to cleave only the nucleic acid oligomer from the nucleic acid oligomer bonded to the solid support of formula (3) and further deprotect it to obtain the nucleic acid oligomer shown by formula (5). Hereinafter, the substituents in each formula will be explained in more detail.

The nucleobase represented by $B^a$ which may be protected with a protecting group and the nucleobase represented by $B^c$ are not particularly limited. Examples of the nucleobase include adenine, cytosine, guanine, uracil, thymine, 5-methylcytosine, pseudouracil, 1-methylpseudouracil and the others. Further, the nucleobase may be substituted with a substituent. Example of the substituents includes a halogen atom such as a fluoro group, a chloro group, a bromo group, and an iodo group; an acyl group such as an acetyl group; an alkyl group such as a methyl group and an ethyl group; and an arylalkyl group such as a benzyl group; an alkoxy group such as a methoxy group; an alkoxyalkyl group such as a methoxy ethyl group; a cyanoalkyl group such as a cyano ethyl group; a hydroxy group; a hydroxyalkyl group; an acyloxy methyl group; an amino group; a monoalkylamino group; a dialkylamino group; a carboxy group; a cyano group; a nitro group; and the combinations of two or more of these substituents.

The protecting group for the nucleobase represented by $B^a$ which may be protected with the protecting group is not particularly limited, known protecting groups used in nucleic acid chemistry can be used, and examples of the protecting groups include a benzoyl group, a 4-methoxybenzoyl group, a 4-methylbenzoyl group, an acetyl group, a propionyl group, a butyryl group, an isobutyryl group, a phenylacetyl group, a phenoxyacetyl group, a 4-tert-butyl phenoxyacetyl group, a 4-isopropyl phenoxyacetyl group, and a (dimethylamino)methylene group, and the others; as well as the combinations of two or more of these protecting groups.

$B^a$ represents more specifically any groups indicated below.

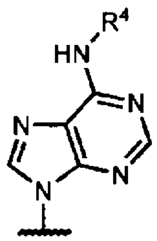
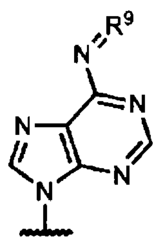
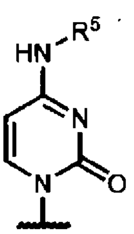
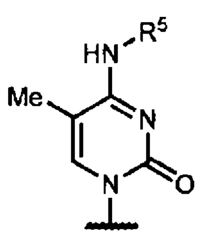

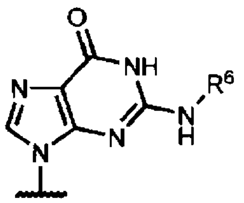 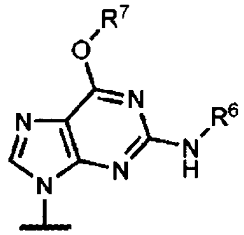 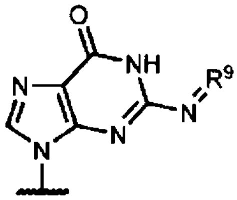 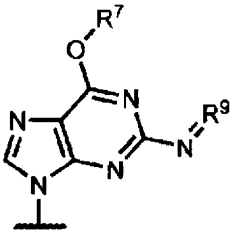

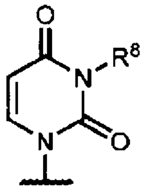 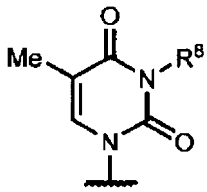 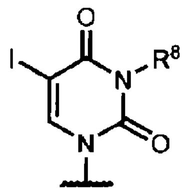 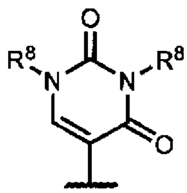

{wherein

$R^4$ represents a hydrogen atom, a methyl group, a phenoxyacetyl group, a 4-tert-butylphenoxyacetyl group, a 4-isopropylphenoxyacetyl group, a phenylacetyl group, an acetyl group, or a benzoyl group,
$R^5$ represents a hydrogen atom, an acetyl group, an isobutyryl group, or a benzoyl group,
$R^6$ represents a hydrogen atom, a phenoxyacetyl group, a 4-tert-butylphenoxyacetyl group, a 4-isoproylphenoxyacetyl group, a phenylacetyl group, an acetyl group or an isobutyryl group,
$R^7$ represents a 2-cyanoethyl group,
$R^8$ represents a hydrogen atom, a methyl group, a benzoyl group, a 4-methoxybenzoyl group, or a 4-methylbenzoyl group, and
$R^9$ represents a dimethylaminomethylene group).

More specifically, $B^c$ includes a group obtained by removing the protecting group from the above specific examples of $B^a$.
$G^1$ and $G^5$ in Fig. 1 (corresponding to $G^1$ defined in formula (I) described herein) are preferably the following groups.

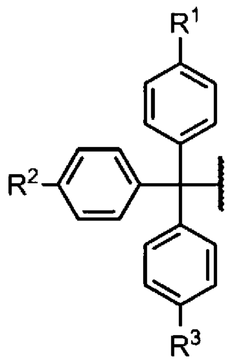

(wherein
$R^1$, $R^2$ and $R^3$, each independently identical to or different from each other, represent a hydrogen atom or an alkoxy group.)
It is preferable that one of $R^1$, $R^2$ and $R^3$ is a hydrogen atom, and the remaining two are alkoxy groups which are identical to or different from each other (preferably identical), and the alkoxy group is particularly preferably a methoxy group. More preferably, $G^5$ is a 4,4'-dimethoxytrityl group (DMTr group).
$G^2$ can be used without any particular limitation as long as it can function as a protecting group for a hydroxy group, and known protecting groups used in amidite compounds can be widely used. Examples of $G^2$ include an alkyl group, an alkenyl group, an alkynyl group, a cycloalkyl group, a haloalkyl group, an aryl group, a heteroaryl group, an arylalkyl group, a cycloalkenyl alkyl group, a cycloalkylalkyl group, a cyclylalkyl group, a hydroxyalkyl group, an aminoalkyl group, an alkoxyalkyl group, a heterocyclylalkenyl group, a heterocyclylalkyl group, a heteroarylalkyl group, a silyl group, a silyloxyalkyl group, a mono, di or tri- alkylsilyl group, a mono, di or tri-alkylsilyloxyalkyl group, and the others, and these groups may be substituted with one or more electron-withdrawing groups.

$G^2$ is preferably an alkyl group substituted with an electron-withdrawing ($E_W$) group. Examples of the electron-withdrawing group include a cyano group, a nitro group, an alkylsulfonyl group, a halogen atom, an arylsulfonyl group, a trihalomethyl group, a trialkylamino group, and the others, and preferably a cyano group.

Particularly preferred as $G^2$ is the following group.

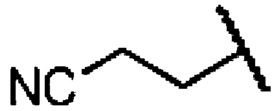

The alkyl group in the definitions of the above $R^1$, $R^2$, $R^3$ and $G^2$ may be a straight chain or a branched chain, and preferably include an alkyl group having 1 to 12 carbon atoms, and more preferably an alkyl group having 1 to 6 carbon atoms. Specific examples of the alkyl group include a methyl group, an ethyl group, a n-propyl group, an isopropyl group, a n-butyl group, an isobutyl group, a tert-butyl group, a n-pentyl group, an isopentyl group, and a hexyl group. The alkyl group moiety constituting the alkoxy group in the definition for above substituents has the same definition as the definition of alkyl group described herein.

In addition, in the method of the present invention, the amidite compound can be used in a free state or a salt state. Examples of the salt of the amidite compound include a base addition salt and an acid addition salt, but which are not particularly limited thereto. Specific example of the base addition salt include salts with inorganic bases such as sodium salts, magnesium salts, potassium salts, calcium salts, and aluminum salts; salts with organic bases such as methylamine, ethylamine, and ethanolamine; salts with basic amino acids such as lysine, ornithine and arginine; and ammonium salts. Specific examples of acid addition salts include mineral acids such as hydrochloric acid, hydrobromic acid, hydroiodic acid, sulfuric acid, nitric acid, phosphoric acid; salts with organic acids such as formic acid, acetic acid, propionic acid, oxalic acid, malonic acid, maleic acid, tartaric acid, fumaric acid, succinic acid, lactic acid, maleic acid, citric acid, methanesulfonic acid, trifluoromethansulfonic acid, ethanesulfonic acid; and salts with acidic amino acids such as aspartic acid and glutamic acid. Examples of the amidite compounds encompass salts, hydrates, solvates, crystal polymorphs, and the others.

R preferably represents a protected hydroxy group. The protecting group when R represents a protected hydroxy group, or the protecting group for the hydroxy group represented by V may be any one which can be used in an amidite method, and for example, a 2'-tert-butyldimethylsilyl (TBS) group, a 2'-bis(2-acetoxyethoxy)methyl (ACE) group, a 2'-(triisopropylsilyloxy)methyl (TOM) group, a 2'-(2-cyanoethoxy)ethyl (CEE) group, a 2'-(2-cyanoethoxy)methyl (CEM) group (described in WO 2006/022323 A), a 2'-para-tolylsulfonylethoxymethyl (TEM) group, a 2'-EMM group (described in WO 2013/027843 A), and a 2'-PMM group (described in WO 2019/208571A) can be used. V is preferably a 2'-tert-butyldimethylsilyl (TBS) group. In addition, when a nucleic acid molecule produced by the method of the present invention is ribonucleoic acid (RNA), and a ribose is included in a nucleic acid molecule, as the protecting group for the hydroxy group at the 2' position of the ribose, a protecting group represented by formula (6) is exemplified as a preferable protecting group. More preferably, a protecting group represented by formula (12) having a cyano group as an electron-withdrawing group represented by $E_W$ is exemplified.

formula (6):

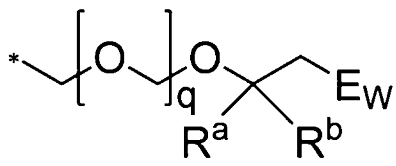

(6)

(wherein

q represents any integer of 0 to 5,

$R^a$ and $R^b$, each independently identical to or different from each other, represent a methyl group, an ethyl group, or a hydrogen atom,

mark * represents the bonding point with the oxygen atom derived from the hydroxy group at the 2' position of the ribose; and

$E_W$ represents an electron-withdrawing group).

formula (12):

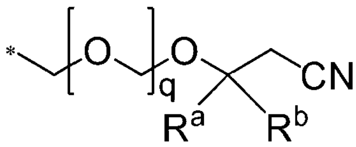

(12)

(wherein
q, $R^a$ and $R^b$ are the same as those defined in formula (6)).

More preferably, in the group represented by formula (12), a group where q is 1 and $R^a$ and $R^b$ are both a hydrogen atom, and a group where q is 1 and either $R^a$ or $R^b$ is a methyl group, and the other is a hydrogen atom are exemplified.

The protecting group represented by formula (6) (including formula (12)) can be synthesized, for example, according to the descriptions in WO 2013/027843 A1 and WO 2019/208571 A1, and the amidite compounds having the protecting group can be used in the production of a nucleic acid compound.

For the nucleic acid elongation reaction, the amidite compound represented by formula (13) shown in Scheme A of Fig. 1 is used.

As the non-nucleotide linker, a linker composed by amino acid backbone (for example, a linker composed by amino acid backbone as described in WO 2006/022323 A1 or WO 2013/027843 A1) is exemplified. Specifically, as a non-limiting example, a linker represented by formula (A14-1), formula (A14-2) or formula (A14-3) (which are described, for example in WO 2019/074110 A1) is exemplified. In addition to these linkers, a linker as described in WO 2012/005368 A1, WO 2018/182008 A1 or WO 2019/074110 A1 is exemplified.

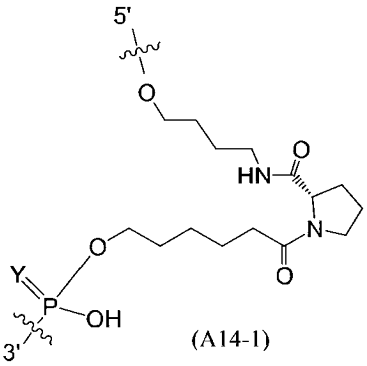

(A14-1) ,

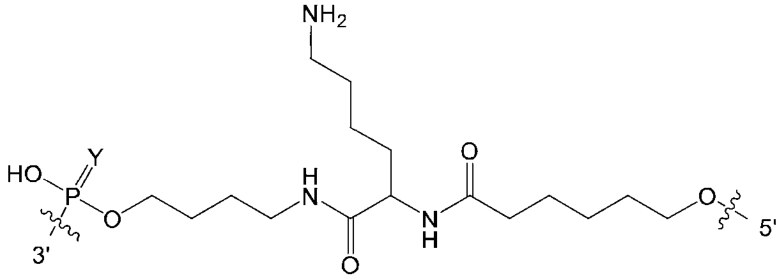

(A14-2)

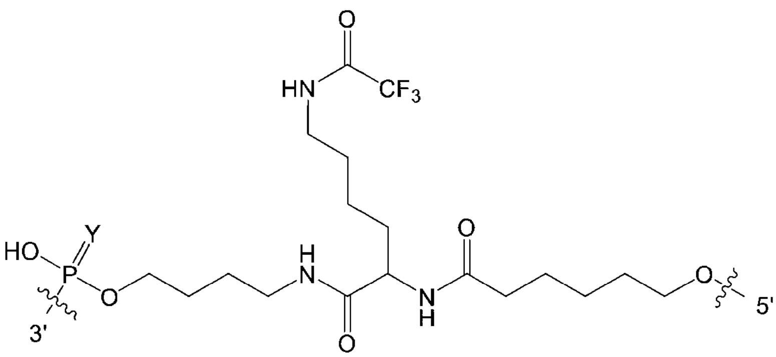

(A14-3)

(wherein Y is the same as described above)

A nucleotide and an amidite wherein an R group in formula (13) and an R' group in formula (5) are substitutes other than a hydroxy group can be also produced from nucleosides which are synthesized according to known methods described in JP 3745226 B2 and so on, or WO 2001/053528 A1, JP 2014-221817 A1 or known methods referred to in these documents. Further, they can be produced by using a commercially available compound in line with the method described in the below Examples or methods with appropriate modifications to these methods.

$G^4$ represents a hydrogen atom, an alkali metal ion, an ammonium ion, an alkyl ammonium ion, or a hydroxyalkyl ammonium ion. Examples of the alkali metal ion include a sodium ion, and a lithium ion. In addition, as the alkylammonium ion, specific examples of alkyl groups include a methyl group, an ethyl group, a n-propyl group, an isopropyl group, a n-butyl group, an isobutyl group, a tert-butyl group, a n-pentyl group, an isopentyl group, and a hexyl group, and specific examples thereof include a diethyl ammonium ion, a triethyl ammonium ion, a tetrabutyl ammonium ion, a hexyl ammonium ion, a dibutyl ammonium ion, and the others. In addition, as the hydroxy alkyl ammonium ion, specific examples of the hydroxyalkyl moiety include hydroxymethyl, hydroxyethyl, hydroxy-n-propyl, hydroxy isopropyl, hydroxy-n-butyl, and tris hydroxy methyl, and more specific examples of the hydroxyalkyl ammonium include tris hydroxymethyl ammonium ion and the others. $G^4$ represents preferably a hydrogen atom.

$G^5$ represents a hydrogen atom or the protecting group for the hydroxy group, and when it represents a protecting group, $G^1$ also represents the same protecting group. When $G^5$ is deprotected, it is a hydrogen atom, and the nucleotide compound in that case is also subjected to a series of nucleic acid elongation reaction steps.

Y is preferably an oxygen atom.

For $W_1$ and $X_1$, preferably, $W_1$ represents an OZ group and $X_1$ represents an R group.

For $W_2$ and $X_2$, preferably, $W_2$ represents a hydroxy group and $X_1$ represents an R group.

For $W_3$ and $X_3$, preferably, each independently represent a hydroxy group.

R' is preferably a hydroxy group.

For the synthesis of the nucleic acid oligomer by the amidite method in the above steps (1) to (5), the nucleic acid elongation reaction can be conducted according to a generally known method (for example, the method described in the above JP 5157168 B2 or JP 5554881 B2) except for the deprotection step relating to the present invention. Hereinafter, each step is described.

(Nucleic acid elongation reaction)

As used herein, "nucleic acid elongation reaction" means that a reaction for elongating oligonucleotide by binding nucleotide sequentially via phosphodiester bond. The nucleic acid elongation reaction can be conducted according to the procedures of the general phosphoramidite method. The nucleic acid elongation reaction may be conducted with a nucleic acid automatic synthesizer and so on which applies the phosphoramidite method.

The chain length (N) of the nucleic acid molecule may be, for example, 20 mer or more (that is, $n \geqq 19$), 40 mer or more (that is, $n \geqq 39$), 50 mer or more (that is, $n \geqq 49$), 60 mer or more (that is, $n \geqq 59$), 80 mer or more (that is, $n \geqq 79$), 100 mer or more (that is, $n \geqq 99$), 200 mer or more (that is, $n \geqq 199$), 2 to 300 mer (that is, $1 \leqq n \leqq 299$), 2 to 250 mer (that is, $1 \leqq n \leqq 249$), 2 to 200 mer (that is, $1 \leqq n \leqq 199$), 10 to 300 mer (that is, $9 \leqq n \leqq 299$), 10 to 250 mer (that is, $9 \leqq n \leqq 249$), 10 to 200 mer (that is, $9 \leqq n \leqq 199$), 10 to 150 mer (that is, $9 \leqq n \leqq 149$), 15 to 300 mer (that is, $14 \leqq n \leqq 299$), 15 to 250 mer (that is, $14 \leqq n \leqq 249$), 15 to 200 mer (that is, $14 \leqq n \leqq 199$), 15 to 150 mer (that is, $14 \leqq n \leqq 149$), or 15 to 110 mer (that is, $14 \leqq n \leqq 109$).

The deprotection step in the step (1) is a step of deprotecting a protecting group for a 5' hydroxy group of an oligonucleotide chain terminal supported on a solid support. As a general protecting group, a 4,4'-dimethoxytrityl group (DMTr group), a 4-monomethoxytrityl group, and a 4,4',4"-trimethoxy trityl group are used. The deprotection can be conducted by using an acid. Examples of the acid for the deprotection include trifluoroacetic acid, dichloroacetic acid, trifluoromethanesulfonic acid, trichloroacetic acid, methanesulfonic acid, hydrochloric acid, acetic acid, p-toluenesulfonic acid, and the others.

The coupling step in the step (2) is a reaction wherein a nucleoside phosphoramidite represented by the following formula (13) as described in Scheme A of Fig. 1 is bonded to the 5' hydroxy group of the oligonucleotide chain terminal deprotected by the deprotection step. Examples of the phosphoramidite to be used in the nucleic acid elongation include formula (13); the uridine EMM amidite described in Example 2 of WO 2013/027843 A1, the cytidine EMM amidite described in Example 3 thereof, the adenosine EMM amidite described in Example 4 thereof, and the guanosine EMM amidite described in Example 5; the uridine PMM amidite, the cytidine PMM amidite, the adenosine PMM amidite, and the guanosine PMM amidite each described in WO 2019/208571 A1. In addition, 2'-OMe, 2'-F, a 2'-O-tert-butyl dimethyl silyl group, a 2'-O-methoxy ethyl group, a 2'-bis(2-acetoxyethoxy)methyl (ACE) group, a 2'-(triisopropylsilyloxy)methyl (TOM) group, a 2'-(2-cyanoethoxy)ethyl (CEE) group, a 2'-(2-cyanoethoxy)methyl (CEM) group, a 2'-para-tolylsulfonylethoxymethyl (TEM) group, 2'-H, and a 2'-fluoro-2'-deoxy-β-D-arabinofuranosyl group. As the nucleoside phosphoramidite, a 5' hydroxy group is protected with a protecting group (for example, DMTr group) is used. The coupling step can be conducted by using an activator which activates the nucleotide phosphoramidite. Examples of the activator include 5-benzylthio-1H-tetrazole (BTT) (referred to as also 5-benzylmercapto-1H-tetrazole), 1H-tetrazole, 4,5-dicyanoimidazole (DCI), 5-ethylthio-1H-tetrazole (ETT), N-methyl benzimidazoliumtriflate (N-MeBIT), benzimidazoliumtriflate (BIT), N-phenylimidazoliumtriflate (N-PhIMT), imidazoliumtriflate (IMT), 5-nitrobenzimidazoliumtriflate (NBT), 1-hydroxybenzotriazole (HOBT), and 5-(bis-3,5-trifluoromethylphenyl)-1H-tetrazole, and the others.

The nucleoside phosphoramidite represented by formula (13) as described in Scheme A of Fig. 1 (hereinafter,

referred to as "amidite") is shown below.

formula:

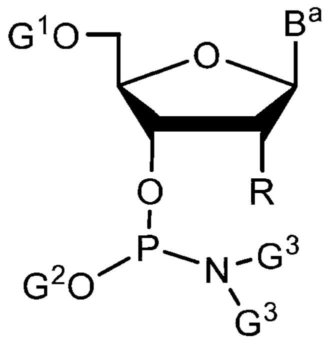

(13)

(wherein
$G^1$, $G^2$, $G^3$, $B^a$, and R are as described above) .

After the coupling step, as needed, the unreacted 5' hydroxy group may be capped. The capping reaction can be conducted by using a known capping solution such as an acetic anhydride-tetrahydrofuran solution, or a phenoxyacetic anhydride / N-methyl imidazole solution.

The oxidation step in the step (3) is a step for converting the phosphite group formed in the coupling step into a phosphate group or a thiophosphate group. This step is a reaction of converting a trivalent phosphorus into a pentavalent phosphorus using an oxidizing agent, which can be conducted by reacting the oxidizing agent with oligonucleic acid derivatives supported on a solid support.

In the case of converting the phosphite group into the phosphate group, as "oxidizing agent", for example, iodine can be used. The oxidizing agent can be used by adjusting a concentration thereof to 0.005 to 2 M. Water can be used as an oxygen source for the oxidation, and pyridine, N-methyl imidazole (NMI), N-methyl morpholine, triethylamine and the other can be used as a base for proceeding with the reaction. In addition, the solvents are not particularly limited as long as they are not involved in the reaction, and can be used as acetonitrile, tetrahydrofuran (THF) or a mixed solvents of these solvents at arbitrary ratio. For example, iodine/water/pyridine/acetonitrile, or iodine/water/pyridine, iodine/water/pyridine/NMI, or iodine/water/pyridine/THF can be used. The reaction temperature is preferably 5°C to 50°C. The reaction time is usually appropriate to be 1 minute to 30 minutes. The amount of the reagent used is preferably 1 to 100 mol, more preferably 1 to 10 mol, based on 1 mol of the compound supported on the solid support.

In the case of converting the phosphite triester group into the thiophosphate group, as "oxidizing agent", for example, sulfur, 3H-1,2-benzodithiol-3-one-1,1-dioxide (Beaucage reagent), 3-amino-1,2,4-dithiazole-5-thione (ADTT), 5-phenyl-3H-1,2,4-dithiazole-3-one (POS), [(N,N-dimethylaminomethyline)amino]-3H-1,2,4-dithiazoline-3-thione (DDTT), and phenylacetyldisulfide (PADS) can be also used. The oxidizing agent can be used by diluting it with an appropriate solvent so as to adjust to 0.001 to 2 M. The solvents to be used in the reaction are not particularly limited as long as they are not involved in the reaction, and include, for example, dichloromethane, acetonitrile, pyridine, or any mixed solvents of these solvents in any arbitrary ratio thereof. The oxidation step may be conducted after the capping operation, or conversely, the capping operation may be conducted after the oxidation step, and the order of the operation is not limited.

In the step (5-1), the protecting group for the hydroxy group at the 5' position of the nucleoside introduced at the end of the elongation may be used for the column purification with the protecting group for the hydroxy group at the 5' position as a tag after cleaving from the solid support and deprotecting the protecting group as described later, or the protecting group of the hydroxy group at the 5' position may be deprotected after the column purification.

In the step (5-2), in the step of deprotecting the phosphate protecting group, after completing the synthesis of a nucleic acid having a desirable sequence, an amine compound is acted to deprotect the protecting group of the phosphate moiety. Examples of the amine compound include diethylamine as described in JP 4705716 B2 and the others.

In the step (5-2), the cleavage of the nucleic acid molecule which is elongated to a desirable chain length on the solid support from the solid support is usually conducted by using a concentrated aqueous ammonium as a cleavage agent.

Further, using ammonia or an amine compound or the others, for example, the oligonucleotide chain is collected by cleaving from the solid support. Examples of the amine compound include methylamine, ethylamine, isopropylamine, ethylenediamine, diethylamine, and the others.

In the step (5-3), the protecting group for the hydroxy group at the 2' position or 3' position of the ribose of the nucleic acid oligomer (4) cleaved from the solid support in the step (5-2) can be removed according to a method described in WO 2006/022323 A1, WO 2013/027843 A1, or WO 2019/208571 A1 to obtain a deprotected nucleic acid oligomer (5).

Examples of the nucleic acid oligomer (oligonucleotide) which can be produced according to the production method of the present invention include those wherein a nucleoside contained in the nucleic acid oligomer is RNA, DNA, as well as RNA having 2'-O-MOE, 2'-O-Me, 2'-F, and LNA, which is not limited thereto. For example, various nucleosides described in Xiulong, Shen et al., Nucleic Acids Research, 2018, Vol. 46, No.46, 1584-1600, and Daniel O'Reilly et al., Nucleic Acids Research, 2019, Vol. 47, No.2, 546-558 are included. Preferably, the nucleic acid oligomer produced by the method of the present invention is ribonucleoic acid (RNA). More preferably, the nucleic acid oligomer produced by the method of the present invention is the nucleic acid oligomer which is a ribonucleoic acid (RNA) wherein the protecting group for the hydroxy group at the 2' position of the ribose is a protecting group represented by formula (6).

By using the production method of the present invention, according to one embodiment, it is possible to produce an oligonucleotide wherein the content of nucleotide deletion product (also referred to as N-1 mer) is reduced.

Here, in the oligonucleotide, the content of N-1 mer to the full length product (FLP (Full Length Product)) in the oligonucleotide, that is, the content (%) of N-1 mer when the full length product (FLP) in the oligonucleotide is taken as 100%, is defined as "Content Ratio of N-1 mer".

Specific examples of the content ratio of N-1 mer in the oligonucleotide include less than 5.8%, 5.7 % or less, 5.5 % or less, 5 % or less, 4.5 % or less, 4 % or less, 3.5 % or less, 3 % or less, 2.5 % or less, 2.3 % or less, 2.3 %, 2.1 % or less, 2.1 %, or also over 0 %, 0.001 % or more, 0.01 % or more, and 0.1 % or more, but which are not limited thereto.

Specific examples of oligonucleotides include the followings, but which are not limited to the followings.

An oligonucleotide wherein the content ratio of N-1 mer in the oligonucleotide is less than 5.8 %.

An oligonucleotide wherein the content ratio of N-1 mer in the oligonucleotide is 5.7 % or less.

An oligonucleotide wherein the content ratio of N-1 mer in the oligonucleotide is 5.5 % or less.

An oligonucleotide wherein the content ratio of N-1 mer in the oligonucleotide is 5 % or less.

An oligonucleotide wherein the content ratio of N-1 mer in the oligonucleotide is 4.5 % or less.

An oligonucleotide wherein the content ratio of N-1 mer in the oligonucleotide is 4 % or less.

An oligonucleotide wherein the content ratio of N-1 mer in the oligonucleotide is 3.5 % or less.

An oligonucleotide wherein the content ratio of N-1 mer in the oligonucleotide is 3 % or less.

An oligonucleotide wherein the content ratio of N-1 mer in the oligonucleotide is 2.5 % or less.

An oligonucleotide wherein the content ratio of N-1 mer in the oligonucleotide is 2.3 % or less.

An oligonucleotide wherein the content ratio of N-1 mer in the oligonucleotide is 2.1 % or less.

An oligonucleotide wherein the chain length of the oligonucleotide is 50 mer or more and the content ratio of N-1 mer in the oligonucleotide is less than 5.8 %.

An oligonucleotide wherein the chain length of the oligonucleotide is 50 mer or more and the content ratio of N-1 mer in the oligonucleotide is 5.7 % or less.

An oligonucleotide wherein the chain length of the oligonucleotide is 50 mer or more and the content ratio of N-1 mer in the oligonucleotide is 5.5 % or less.

An oligonucleotide wherein the chain length of the oligonucleotide is 50 mer or more and the content ratio of N-1 mer in the oligonucleotide is 5 % or less.

An oligonucleotide wherein the chain length of the oligonucleotide is 50 mer or more and the content ratio of N-1 mer in the oligonucleotide is 4.5 % or less.

An oligonucleotide wherein the chain length of the oligonucleotide is 50 mer or more and the content ratio of N-1 mer in the oligonucleotide is 4 % or less.

An oligonucleotide wherein the chain length of the oligonucleotide is 50 mer or more and the content ratio of N-1 mer in the oligonucleotide is 3.5 % or less.

An oligonucleotide wherein the chain length of the oligonucleotide is 50 mer or more and the content ratio of N-1 mer in the oligonucleotide is 3 % or less.

An oligonucleotide wherein the chain length of the oligonucleotide is 50 mer or more and the content ratio of N-1 mer in the oligonucleotide is 2.5 % or less.

An oligonucleotide wherein the chain length of the oligonucleotide is 50 mer or more and the content ratio of N-1 mer in the oligonucleotide is 2.3 % or less.

An oligonucleotide wherein the chain length of the oligonucleotide is 50 mer or more and the content ratio of N-1 mer in the oligonucleotide is 2.1 % or less.

An oligonucleotide wherein the chain length of the oligonucleotide is 50 mer to 200 mer and the content ratio of N-1 mer in the oligonucleotide is less than 5.8 %.

An oligonucleotide wherein the chain length of the oligonucleotide is 50 mer to 200 mer and the content ratio of N-1 mer in the oligonucleotide is 5.7 % or less.

An oligonucleotide wherein the chain length of the oligonucleotide is 50 mer to 200 mer and the content ratio of N-1 mer in the oligonucleotide is 5.5 % or less.

An oligonucleotide wherein the chain length of the oligonucleotide is 50 mer to 200 mer and the content ratio of N-1 mer in the oligonucleotide is 5 % or less.

An oligonucleotide wherein the chain length of the oligonucleotide is 50 mer to 200 mer and the content ratio of N-1 mer in the oligonucleotide is 4.5 % or less.

An oligonucleotide wherein the chain length of the oligonucleotide is 50 mer to 200 mer and the content ratio of N-1 mer in the oligonucleotide is 4 % or less.

An oligonucleotide wherein the chain length of the oligonucleotide is 50 mer to 200 mer and the content ratio of N-1 mer in the oligonucleotide is 3.5 % or less.

An oligonucleotide wherein the chain length of the oligonucleotide is 50 mer to 200 mer and the content ratio of N-1 mer in the oligonucleotide is 3 % or less.

An oligonucleotide wherein the chain length of the oligonucleotide is 50 mer to 200 mer and the content ratio of N-1 mer in the oligonucleotide is 2.5 % or less.

An oligonucleotide wherein the chain length of the oligonucleotide is 50 mer to 200 mer and the content ratio of N-1 mer in the oligonucleotide is 2.3 % or less.

An oligonucleotide wherein the chain length of the oligonucleotide is 50 mer to 200 mer and the content ratio of N-1 mer in the oligonucleotide is 2.1 % or less.

An oligonucleotide wherein the chain length of the oligonucleotide is 50 mer to 250 mer and the content ratio of N-1 mer in the oligonucleotide is less than 5.8 %.

An oligonucleotide wherein the chain length of the oligonucleotide is 50 mer to 250 mer and the content ratio of N-1 mer in the oligonucleotide is 5.7 % or less.

An oligonucleotide wherein the chain length of the oligonucleotide is 50 mer to 250 mer and the content ratio of N-1 mer in the oligonucleotide is 5.5 % or less.

An oligonucleotide wherein the chain length of the oligonucleotide is 50 mer to 250 mer and the content ratio of N-1 mer in the oligonucleotide is 5 % or less.

An oligonucleotide wherein the chain length of the oligonucleotide is 50 mer to 250 mer and the content ratio of N-1 mer in the oligonucleotide is 4.5 % or less.

An oligonucleotide wherein the chain length of the oligonucleotide is 50 mer to 250 mer and the content ratio of N-1 mer in the oligonucleotide is 4 % or less.

An oligonucleotide wherein the chain length of the oligonucleotide is 50 mer to 250 mer and the content ratio of N-1 mer in the oligonucleotide is 3.5 % or less.

An oligonucleotide wherein the chain length of the oligonucleotide is 50 mer to 250 mer and the content ratio of N-1 mer in the oligonucleotide is 3 % or less.

An oligonucleotide wherein the chain length of the oligonucleotide is 50 mer to 250 mer and the content ratio of N-1 mer in the oligonucleotide is 2.5 % or less.

An oligonucleotide wherein the chain length of the oligonucleotide is 50 mer to 250 mer and the content ratio of N-1 mer in the oligonucleotide is 2.3 % or less.

An oligonucleotide wherein the chain length of the oligonucleotide is 50 mer to 250 mer and the content ratio of N-1 mer in the oligonucleotide is 2.1 % or less.

An oligonucleotide wherein the chain length of the oligonucleotide is 50 mer to 300 mer and the content ratio of N-1 mer in the oligonucleotide is less than 5.8 %.

An oligonucleotide wherein the chain length of the oligonucleotide is 50 mer to 300 mer and the content ratio of N-1 mer in the oligonucleotide is 5.7 % or less.

An oligonucleotide wherein the chain length of the oligonucleotide is 50 mer to 300 mer and the content ratio of N-1 mer in the oligonucleotide is 5.5 % or less.

An oligonucleotide wherein the chain length of the oligonucleotide is 50 mer to 300 mer and the content ratio of N-1 mer in the oligonucleotide is 5 % or less.

An oligonucleotide wherein the chain length of the oligonucleotide is 50 mer to 300 mer and the content ratio of N-1 mer in the oligonucleotide is 4.5 % or less.

An oligonucleotide wherein the chain length of the oligonucleotide is 50 mer to 300 mer and the content ratio of N-1 mer in the oligonucleotide is 4 % or less.

An oligonucleotide wherein the chain length of the oligonucleotide is 50 mer to 300 mer and the content ratio of N-1 mer in the oligonucleotide is 3.5 % or less.

An oligonucleotide wherein the chain length of the oligonucleotide is 50 mer to 300 mer and the content ratio of N-1 mer in the oligonucleotide is 3 % or less.

An oligonucleotide wherein the chain length of the oligonucleotide is 50 mer to 300 mer and the content ratio of N-1 mer in the oligonucleotide is 2.5 % or less.
An oligonucleotide wherein the chain length of the oligonucleotide is 50 mer to 300 mer and the content ratio of N-1 mer in the oligonucleotide is 2.3 % or less.
An oligonucleotide wherein the chain length of the oligonucleotide is 50 mer to 300 mer and the content ratio of N-1 mer in the oligonucleotide is 2.1 % or less.
An oligonucleotide wherein the chain length of the oligonucleotide is 100 mer to 300 mer and the content ratio of N-1 mer in the oligonucleotide is less than 5.8 %.
An oligonucleotide wherein the chain length of the oligonucleotide is 100 mer to 300 mer and the content ratio of N-1 mer in the oligonucleotide is 5.7 % or less.
An oligonucleotide wherein the chain length of the oligonucleotide is 100 mer to 300 mer and the content ratio of N-1 mer in the oligonucleotide is 5.5 % or less.
An oligonucleotide wherein the chain length of the oligonucleotide is 100 mer to 300 mer and the content ratio of N-1 mer in the oligonucleotide is 5 % or less.
An oligonucleotide wherein the chain length of the oligonucleotide is 100 mer to 300 mer and the content ratio of N-1 mer in the oligonucleotide is 4.5 % or less.
An oligonucleotide wherein the chain length of the oligonucleotide is 100 mer to 300 mer and the content ratio of N-1 mer in the oligonucleotide is 4 % or less.
An oligonucleotide wherein the chain length of the oligonucleotide is 100 mer to 300 mer and the content ratio of N-1 mer in the oligonucleotide is 3.5 % or less.
An oligonucleotide wherein the chain length of the oligonucleotide is 100 mer to 300 mer and the content ratio of N-1 mer in the oligonucleotide is 3 % or less.
An oligonucleotide wherein the chain length of the oligonucleotide is 100 mer to 300 mer and the content ratio of N-1 mer in the oligonucleotide is 2.5 % or less.
An oligonucleotide wherein the chain length of the oligonucleotide is 100 mer to 300 mer and the content ratio of N-1 mer in the oligonucleotide is 2.3 % or less.
An oligonucleotide wherein the chain length of the oligonucleotide is 100 mer to 300 mer and the content ratio of N-1 mer in the oligonucleotide is 2.1 % or less.
As typical examples of nucleic acid molecule which can be used in the production method of the present invention, the following examples are indicated in addition to examples described in working examples, but which are not limited thereto.
Hereinafter, in the description of sequences, U represents uridine, C represents cytidine, A represents adenosine, and G represents guanosine.
The nucleic acid molecules having the following sequences (A) and (B) as described in WO 2019/060442 A1 is exemplified.

Sequence (A): 5'-AUGGAAUmACUCUUGGUUmACdTdT-3' (expressed according to a ST.25 format) (5'-ATGGAATmACTCTTGGTTmACdTdT-3' (expressed according to a ST.26 format)) (Antisense) (Sequence No. 1) 21 mer
Sequence (B): 5'-GUmAACmCmAAGAGUmAUmUmCmCmAUmdTdT (expressed according to a ST.25 format) (5'-GTmAACmCmAAGAGTmATmTmCmCmATmdTdT-3' (expressed according to a ST.26 format)) (Sense) (Sequence No. 2) 21 mer

In the sequences (A) and (B), Um represents 2'-O-methyluridine (expressed according to a ST.25 format), Tm represents 2'-O-methyluridine (expressed according to a ST.26 format), Cm represents 2'-O-methylcytidine, and dT represents thymidine. As described herein, unless stated otherwise, the abbreviations in the sequence may be applied to both of the ST.25 format and the ST.26 format.
The nucleic acid molecule as described in Daniel O'Reilly et al., Nucleic Acids Research, 2019, Vol. 47, No.2, 546-558 (refer to p. 553) is exemplified. Typical examples thereof include the nucleic acid molecule having the following sequence (C).
Sequence (C): 5'-AGAGCCAGCCUUCUUAUUGUUUUAGAGCUAUGCUGU-3' (expressed according to a ST.25 format) (5'-AGAGCCAGCCTTCTTATTGTTTTAGAGCTATGCTGT-3' (expressed according to a ST.26 format)) (Sequence No. 3) 36 mer
The nucleic acid molecule having the following sequence (D) as described in Nucleic Acids Research, 2019, Vol. 47, No. 2: 547 is exemplified.
Sequence (D): 5'-ACAGCAUAGCAAGUUAAAAUAAGGCUAGUCCGUUAUCAACUUGAAAAAGUGGCACCGAG UCGGUGCU-3' (expressed according to a ST.25 format) ((5'-ACAGCATAGCAAGTTAAAATAAGGCTAGTCCGTTATC AACTTGAAAAAGTGGCACCGAG TCGGTGCT-3' (expressed according to a ST.26 format)) (Sequence No. 4) 67 mer
The nucleic acid molecule as described in JP 2015-523856 A1 page 173 is exemplified. Typical examples thereof

include the nucleic acid molecule having the following sequence (E).
Sequence (E): 5'-GUUUUCCCUUUUCAAAGAAAUCUCCUGGGCACCUAUCUUCUUAGGUGCCCUCCCUUGUU UAAACCUGACCAGUUAACCGGCUGGUUAGGUUUUU-3' (expressed according to a ST.25 format) ((5'-GTTTTCCC TTTTCAAAGAAATCTCCTGGGCACCTATCTTCTTAGGTGCCCTCCCTTGTT TAAACCTGACCAGTTAACCGGCTGG TTAGGTTTT-3' (expressed according to a ST.26 format)) (Sequence No. 5) 94 mer

The nucleic acid molecules as described in JP 2017-537626 A1 are exemplified. Typical examples thereof include the nucleic acid molecules having the following sequences (F), (G), (H) and (J) .

Sequence (F): 5'-AGUCCUCAUCUCCCUCAAGCGUUUUAGAGCUAGUAAUAGCAAGUUAAAAUAAGGCUAGU CCGUUAUCAACUUGAAAAAGUGGCACCGAGUCGGUGCUUUU-3' (expressed according to a ST.25 format) ((5'-AGTCCTCATCTCCCTCAAGCGTTTTAGAGCTAGTAATAGCAAGTTAAAATAAGGCTAGT CCGTTATCAACTTGA AAAAGTGGCACCGAGTCGGTGCTTTT-3' (expressed according to a ST.26 format)) (Sequence No. 6) 100 mer
Sequence (G): 5'-GCAGAUGUAGUGUUUCCACAGUUUAAGAGCUAUGCUGGAAACAGCAUAGCAAGUUUAAA UAAGGCUAGUCCGUUAUCAACUUGAAAAAGUGGCACCGAGUCGGUGCUUUUUUU-3' (expressed according to a ST.25 format) ((5'-GCAGATGTAGTGTTTCCACAGTTTAAGAGCTATGCTGGAAACAGCATAGCAAGTTTAAA TAAGGCTAGTCCGTTATCAACTTGAAAAAGTGGCACCGAGTCGGTGCTTTTTTT-3' (expressed according to a ST.26 format)) (Sequence No. 7) 113 mer
Sequence (H): 5'-dAdGdTdCdCdTdCdAdTdCdTdCdCdCdTdCdAdAdGdCGUUUAAGAGCUAUGCUGGU AACA GCAUAGCAAGUUUAAAUAAGGCUAGUCCGUUAUCAACUUGAAAAAGUGGCACCGA GUCGGUG-CUUUUUUU-3' (expressed according to a ST.25 format) ((5'-dAdGdTdCdCdTdCdAdTdCdTdCdCdCdTdCdAdAd GdCGTTTAAGAGCTATGCTGGT AACAGCATAGCAAGTTTAAATAAGGCTAGTCCGTTATCAACTTGAAAAAGT GGCACCGA GTCGGTGCTTTTTTT-3' (expressed according to a ST.26 format) (Sequence No. 8) 113 mer

In the sequence (H), dT represents thymidine, dC represents 2'-deoxycytidine, dA represents 2'-deoxyadenosine, and dG represents 2'-deoxyguanosine.
Sequence (J): 5'-AmsGmsUmsCCUCAUCUCCCUCAAGCGUUUAAGAGCUAUGCUGGUAACAGCAUAGCAAG UU UAAAUAAGGCUAGUCCGUUAUCAACUUGAAAAAGUGGCACCGAGUCGGUGCUUUUms UmsUmsU-3' (expressed according to a ST.25 format) ((5'-AmsGmsTmsCCTCATCTCCCTCAAGCGTTTAAGAGCTATGCTGGTAACA GCATAGCAAG TTTAAATAAGGCTAGTCCGTTATCAACTTGAAAAAGTGGCACCGAGTCGGTGCTTTTms TmsTmsT-3' (expressed according to a ST.26 format)) (Sequence No. 9) 113 mer

In the sequence (J), "Um" represents 2'-O-methyluridine (expressed according to a ST.25 format), "Tm" represents 2'-O-methyluridine (expressed according to a ST.26 format), "Am" represents 2'-O-methyladenosine, "Gm" represents 2'-O-methylguanosine, and "s" represents phosphorothioate modification.

EXAMPLES

Hereinafter, the present invention is explained in more detail by working examples, and the present invention is not limited to these working examples.

Measurement Method

Firstly, various measurement methods used in the following tests are shown below.

(Measurement method 1: Measurement of FLP purity and N-1 mer content in the oligonucleotide)

The purity of oligonucleotide was measured with the use of HPLC.
FLP represents the product which is a full chain length (Full Length Product).
The HPLC measurement conditions are shown in Table 1 below.

[Table 1]

| | |
|---|---|
| Column | DNAPac™ PA200 4 × 250 mm |
| Flow rate | 1.0 mL/min |
| Detection wavelength | 260 nm |
| Mobile phase A | 25 mM Tris-HCl buffer (pH=8.0), 10% $CH_3CN$, 6M Urea water |
| Mobile phase B | 500 mM $NaClO_4$, 25 mM Tris-HCl buffer (pH=8.0), 10% $CH_3CN$, 6M Urea water |

(continued)

| | |
|---|---|
| Gradient condition | Bconc. 20%(0 min)-60%(60 min)-90%(60.01 min)-90%(65 min)-20%(65.01 min)-20%(80 min) |
| Column temperature | 80°C |
| Injection volume | 10 μL |

The N-1 mer content represents the content of N-1 mer (area percentage) in the obtained oligonucleotide which is calculated by analyzing the obtained oligonucleotide according to the measurement method 1. The FLP purity represents the content of FLP (area percentage) in the obtained oligonucleotide which is calculated by analyzing the obtained oligonucleotide according to the measurement method 1.

(Measurement method 2: Measurement of oligonucleotide yield)

$OD_{260}$ of the crude product was measured. $OD_{260}$ represents the absorbance at UV260 nm per 10 mm optical path length in a 1 mL solution (pH = 7.5). Since it is generally known that 1 $OD_{260}$ = 40 μg for RNA, the yield was calculated based on the measured value of $OD_{260}$

<Solid-phase synthesis of oligonucleotides)

Sequence (I): 5'-UmUmUmUmUmUmUmUmUmUmUmUmUmUmUmUmUmUmUmUmUmUmUm UmUmU mUmUmUmUmUmUmUmUmUmUmUmUmUmUmUmUmUmUmUm-3' (expressed according to a ST.25 format)
(5'-TmTmTmTmTmTmTmTmTmTmTmTmTmTmTmTmTmTmTmTmTmTmTmTmTmTmTmTmT mTmTmTmTmTmTmTmTmTmTmTmTmTmTmTmTmTmTmTmTm-3') (expressed according to a ST.26 format) (Sequence No. 10) 50 mer

"Um" represents 2'-O-methyluridine (expressed according to a ST.25 format), and "Tm" is 2'-O-methyluridine (expressed according to a ST.26 format) in the sequence (I). As described herein, unless stated otherwise, the abbreviations in the sequence may be applied to both of the ST.25 format and the ST.26 format.

The sequence (I) represents the structure (16) below.

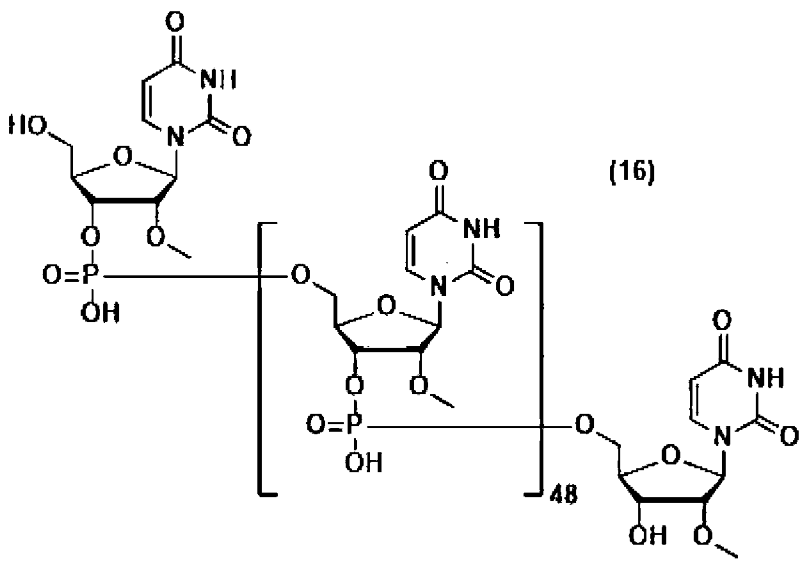

(16)

Using Controlled Pore Glass (CPG) as a solid support and NTS M-4MX-E (manufactured by NIHON TECHNO SERVICE CO. LTD) as a nucleic acid synthesizer, according to the phosphoramidite solid-phase synthesis method, an oligonucleotide composed by sequence (I) was synthesized from the 3' side to the 5' side. The synthesis was conducted on a scale of about 1 μmol scale. In addition, in the synthesis, 2'-OMe-U amidite represented by formula (18) was used, and a

high-purity solution of dichloroacetic acid in toluene was used as a deblocking solution, and a 5-benzylthio-1H-tetrazole solution was used as a coupling agent, and an iodine solution was used as an oxidizing agent, and a phenoxyacetic anhydride solution and a N-methyl imidazole solution were used as a capping solution,

Next, specific production examples of the oligonucleotide produced by the production method of the present invention are shown. Here, in the following examples, the oligonucleotide produced according to the production method of the present invention is the oligonucleotide having the sequence (I) shown in the sequence No. 10.

In addition, the CPG on which 2'-OMe-U derivatives is supported, which is described in the following Examples and Comparative Examples, represents the compound represented by the following formula (17). However, a circle as depicted in formula (17) represents CPG schematically.

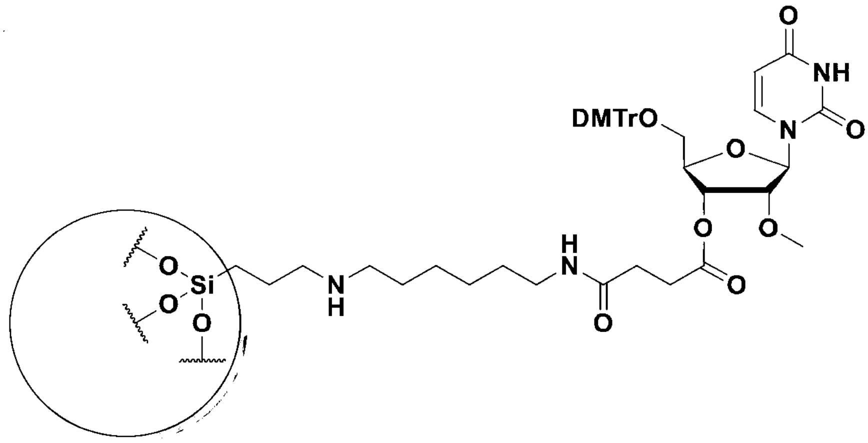

(17)

Example 1

Using Controlled Pore Glass (CPG) on which 1.01 μmol of 2'-OMe-U derivative was supported, and 2'-OMe-U amidite represented by formula (18), the oligonucleotide represented by sequence (I) was automatically synthesized by NTS M-4MX-E (manufactured by NIHON TECHNO SERVICE CO. LTD) from the 3'-side to the 5'-side. In the automatic synthesis procedure, firstly a deblocking solution (1-dodecanethiol : dichloroacetic acid : toluene = 0.5 : 3.0 : 96.5 was liquid-transferred to CPG to deprotect the trityl protecting group at the 5' position. Subsequently, 2'-OMe-U amidite and 5-benzylmercapto-1H-tetrazole as a coupling agent were liquid-transferred to CPG to allow a coupling reaction to proceed for the hydroxy group at the 5'-position. Subsequently, an oxidizing solution containing 50 mM iodine was liquid-transferred to convert the phosphite group into the phosphate group. Subsequently, a 0.1 M phenoxy acetic anhydride solution in acetonitrile and a 10% N-methyl imidazole / 10% 2,6-lutidine solution in acetonitrile were used as a capping solution, thereby a capping was conducted at the reaction point where the coupling did not proceed. Further, after repeating these steps 49 times in total, the protecting group (DMTr group) in the base at the 5'-terminal was deprotected with a deblocking solution (1-dodecanethiol / dichloroacetic acid / toluene = 0.5 / 3.0 / 96.5) and the oligonucleotide of the sequence represented by sequence (I) was synthesized on the CPG support. After that, 750 μL of 28% aqueous ammonia and 250 μL of ethanol were transferred to the CPG support on which 1.01 μmol of the oligonucleotide was supported, and the resulting mixture was kept at 40°C for 4 hours to release the oligonucleotide from the solid support. After that, the solid support was removed by filtration, and aqueous ammonia and ethanol were removed by drying under reduced pressure to obtain a desirable oligonucleotide as a dried solid. As a result of measuring the purity of the oligonucleotide for the obtained product using the method described in the measurement method 1, the content of N-1 mer was 1.5 %, and the purity of FLP was 71.3 %. In addition, when the yield of the oligonucleotide was measured using the method described in the measurement method 2, the yield was 12.6 mg, and when the value was converted to the yield per CPG on which 1.00 μmol of 2'-OMe-U derivative was supported, it was 12.5 mg.

The result was shown in Table 2.

2'-OMe-U amidite represented by formula (18) is shown as the following structure.

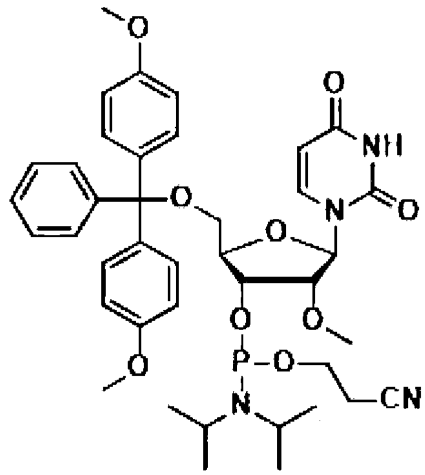

(18)

Example 2

The oligonucleotide of sequence (I) was obtained in the same way as in Example 1 except for using cyclohexanethiol / dichloroacetic acid / toluene = 0.5 / 3.0 / 96.5 as a deblocking solution. As a result of measuring the purity of the oligonucleotide using the method described in the measurement method 1, the content of N-1 mer was 1.6 %, and the purity of FLP was 70.4 %. In addition, when the yield of the oligonucleotide was measured using the method described in the measurement method 2, the yield was 12.6 mg, and when the value was converted to the yield per CPG on which 1.00 μmol of 2'-OMe-U derivative was supported, it was 12.5 mg.

The result was shown in Table 2.

Comparative Example 1

The oligonucleotide of sequence (I) was obtained in the same way as in Example 1 except for using the CPG on which 1.00 μmol of 2'-OMe-U derivative was supported and dichloroacetic acid / toluene = 3.0 / 97.0 as a deblocking solution. As a result of measuring the purity of the oligonucleotide using the method described in the measurement method 1, the content of N-1 mer was 4.9 %, and the purity of FLP was 62.8 %. In addition, when the yield of the oligonucleotide was measured using the method described in the measurement method 2, the yield was 12.2 mg.

The result was shown in Table 2.

Comparative Example 2

The oligonucleotide of sequence (I) was obtained in the same way as in Example 1 except for using the CPG on which 0.96 μmol of 2'-OMe-U derivative was supported and triethylsilane / dichloroacetic acid / toluene = 3.0 /3.0 / 94.0 as a deblocking solution. As a result of measuring the purity of the oligonucleotide using the method described in the measurement method 1, the content of N-1 mer was 3.8 %, and the purity of FLP was 65.7 %. In addition, when the yield of the oligonucleotide was measured using the method described in the measurement method 2, the yield was 11.7 mg, and when the value was converted to the yield per CPG on which 1.00 μmol of 2'-OMe-U derivative was supported, it was 12.2 mg.

The result was shown in Table 2.

Comparative Example 3

The oligonucleotide of sequence (I) was obtained in the same way as in Example 1 except for using the CPG on which 1.03 μmol of 2'-OMe-U derivative was supported and methanol / dichloroacetic acid / toluene = 0.5 /3.0 / 96.5 as a deblocking solution. As a result of measuring the purity of the oligonucleotide using the method described in the measurement method 1, the content of N-1 mer was 9.6 %, and the purity of FLP was 63.5 %. In addition, when the yield of the oligonucleotide was measured using the method described in the measurement method 2, the yield was 12.7 mg, and when the value was converted to the yield per CPG on which 1.00 μmol of 2'-OMe-U derivative was supported, it was 12.3 mg.

The result was shown in Table 2.

Comparative Example 4

The oligonucleotide of sequence (I) was obtained in the same way as in Example 1 except for using the CPG on which 1.04 μmol of 2'-OMe-U derivative was supported and isopropylalcohol / dichloroacetic acid / toluene = 0.5 /3.0 / 96.5 as a deblocking solution. As a result of measuring the purity of the oligonucleotide using the method described in the measurement method 1, the content of N-1 mer was 12.5 %, and the purity of FLP was 59.2 %. In addition, when the yield of the oligonucleotide was measured using the method described in the measurement method 2, the yield was 12.4 mg, and when the value was converted to the yield per CPG on which 1.00 μmol of 2'-OMe-U derivative was supported, it was 11.9 mg.

The result was shown in Table 2.

The results of Examples 1 and 2, as well as Comparative Examples 1 to 4 are shown in Table 2.

Table 2

| | Cation scavenger | Yield (mg/μmol) | N-1 mer content (%) | FLP purity (%) | N-1 mer /FLP (%) |
|---|---|---|---|---|---|
| Example 1 | 1-dodecane thiol | 12.5 | 1.5 | 71.3 | 2.1 |
| Example 2 | cyclohexane thiol | 12.5 | 1.6 | 70.4 | 2.3 |
| Comparative Example 1 | - | 12.2 | 4.9 | 62.8 | 7.8 |
| Comparative Example 2 | triethylsilane | 12.2 | 3.8 | 65.7 | 5.8 |
| Comparative Example 3 | methanol | 12.3 | 9.6 | 63.5 | 15.1 |
| Comparative Example 4 | isopropanol | 11.9 | 12.5 | 59.2 | 21.1 |

In Table 2, N-1 mer content represents the content of N-1 mer (area percentage) in the obtained oligonucleotide which is calculated by analyzing the obtained oligonucleotide according to the measurement method 1. In addition, the FLP purity represents the content of FLP (area percentage) in the obtained oligonucleotide which is calculated by analyzing the obtained oligonucleotide according to the measurement method 1. "N-1 mer / FLP" represents the content ratio of N-1 mer when the content of FLP in the obtained oligonucleotide is deemed 100 %, which is calculated by the following equation.

"N-1 mer / FLP" (%) = N-1 mer content / FLP purity $\times$ 100

(INDUSTRIAL APPLICABILITY)

The present invention provides an efficient deprotection reaction of the protecting group for the 5' hydroxy group using a thiol compound as a cation scavenger. In addition, an improvement in the yield and purity of an oligonucleotide produced according to the production method for an oligonucleotide can be expected.

(FREE TEXT OF SEQUENCE LISTING)

Sequence Nos. 1 to 10 in Sequence Listing represent a base sequence of oligonucleotides produced according to the production method of the present invention.

(SEQUENCE LISTING)

## Claims

1. A production method for an oligonucleotide by solid-phase synthesis method, which comprises reacting an oligonucleotide wherein the hydroxy group at the 5' terminal is protected with a protecting group that can be removed under acidic conditions **characterised in that** the reaction is performed with an acid in the presence of a thiol to remove the protecting group for the hydroxy group at the 5' terminal.

2. The production method according to claim 1 wherein the thiol is a C2-C20 alkyl thiol, or a C4-C8 cycloalkyl thiol.

3. The production method according to claim 1 or 2 wherein the thiol is 1-dodecanethiol or cyclohexanethiol.

4. The production method according to any one of claims 1 to 3 wherein the protecting group for the hydroxy group at the 5' terminal is a protecting group represented by the following formula:

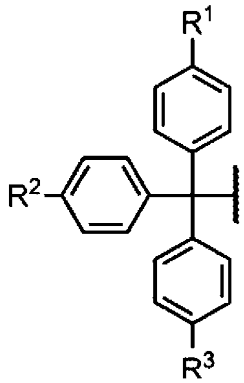

[wherein $R^1$, $R^2$ and $R^3$, each independently identical to or different from each other, represent a hydrogen atom or an alkoxy group).

5. The production method according to any one of claims 1 to 4 wherein the protecting group for the hydroxy group at the 5' terminal is a 4,4'-dimethoxytrityl group (DMTr group).

6. The production method according to any one of claims 1 to 5 wherein the acid is trifluoroacetic acid, dichloroacetic acid, trifluoromethanesulfonic acid, trichloroacetic acid, methanesulfonic acid, hydrochloric acid, acetic acid, or p-toluenesulfonic acid.

7. The production method according to any one of claims 1 to 5 wherein the acid is dichloroacetic acid.

8. The production method according to claim 7 wherein the acid is dichloroacetic acid in the co-existence of aprotic inert solvent having a lower boiling point than dichloroacetic acid.

9. The production method according to claim 8 wherein the aprotic inert solvent having a lower boiling point than dichloroacetic acid is one or more solvents selected from dichloromethane, acetonitrile, or an aromatic organic solvent.

10. The production method according to claim 9 wherein the aromatic organic solvent is toluene.

11. The production method according to claim 7 wherein the dichloroacetic acid is dichloroacetic acid wherein the molar ratio of formaldehyde to dichloroacetic acid is $81 \times 10^{-5}$ or less, and the molar ratio of dichloroacetic anhydride to dichloroacetic acid is $20 \times 10^{-5}$ or less.

12. The production method according to any one of claims 1 to 11, wherein the oligonucleotide wherein the hydroxy group at the 5' terminal is protected with the protecting group that can be removed under acidic conditions is an oligonucleotide represented by formula (1):

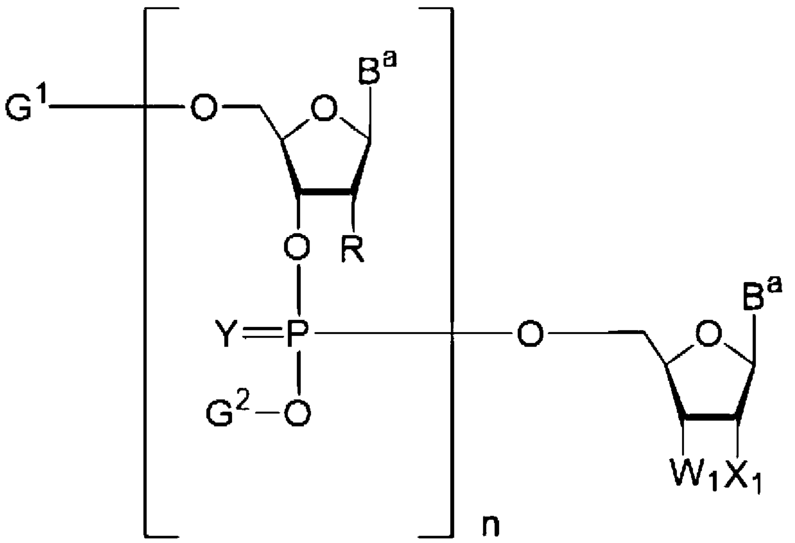

(1)

(wherein

$G^1$ represents a protecting group for a hydroxy group,
$G^2$ represents a protecting group for a hydroxy group,
$B^a$ represents a nucleobase, each independently identical to or different from each other, which may be protected with a protecting group,
R, each independently identical to or different from each other, represents a protected hydroxy group, a hydrogen atom, a fluorine atom, a methoxy group, a 2-methoxyethyl group, or an OQ' group,
Q', each independently identical to or different from each other, represents a methylene group which is bonded to the carbon atom at the 4' position of the ribose, an ethylene group which is bonded to the carbon atom at the 4' position of the ribose, or an ethylidene group which is bonded to the carbon atom at the 4' position of the ribose,
Y, each independently identical to or different from each other, represents an oxygen atom or a sulfur atom,
n represents any integer of 1 to 300,
$W_1$ represents an OZ group and $X_1$ represents an R group, or
$W_1$ represents an OV group and $X_1$ represents an OZ group,
V represents a protecting group for a hydroxy group,
Z is a group having a structure consisting of a solid support and a connecting group, and
when n is an integer of 2 or more, a non-nucleotide linker may be incorporated between respective nucleotides in the nucleic acid molecule represented by formula (1)), and
the nucleotide wherein the protecting group for the hydroxy group at the 5' terminal is removed is an oligonucleotide represented by formula (2):

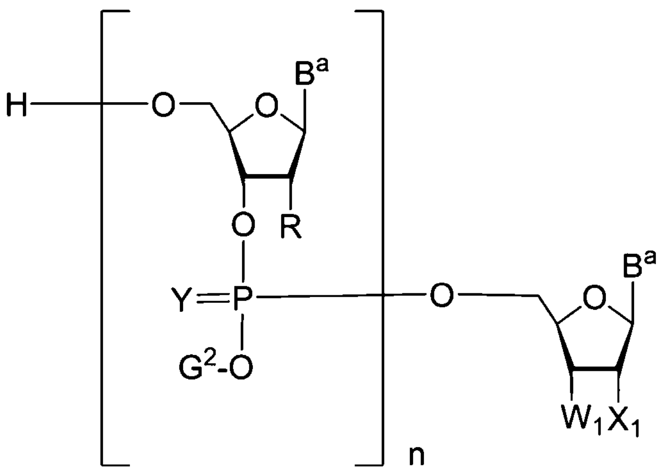

(2)

(wherein

$G^2$, $B^a$, R, Y, $X_1$, $W_1$ and n are as described above, and
as defined in formula (1), a non-nucleotide linker may be incorporated between nucleotides).

**13.** The production method according to claim 12, wherein the oligonucleotide wherein the hydroxy group at the 5' terminal is protected with the protecting group that can be removed under acidic conditions is an oligonucleotide represented by formula (1'):

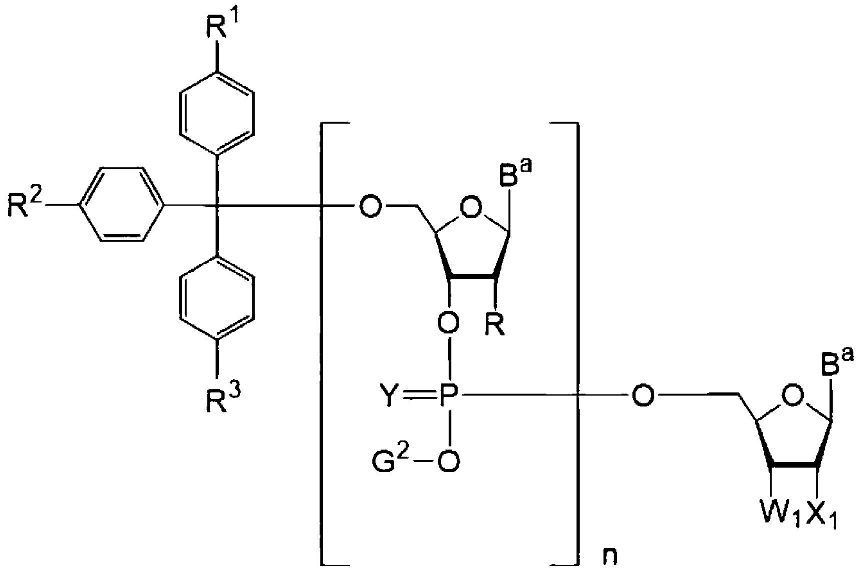

(1')

(wherein

$G^2$, $B^a$, R, Y, $X_1$, $W_1$ and n are as described in claim and
$R^1$, $R^2$ and $R^3$, each independently identical to or different from each other, represent a hydrogen atom or an alkoxy group).

**14.** The production method according to claim 13 wherein $R^1$ and $R^2$ are methoxy groups, and $R^3$ is a hydrogen atom.

**15.** A production method for an oligonucleotide represented by formula (2'):

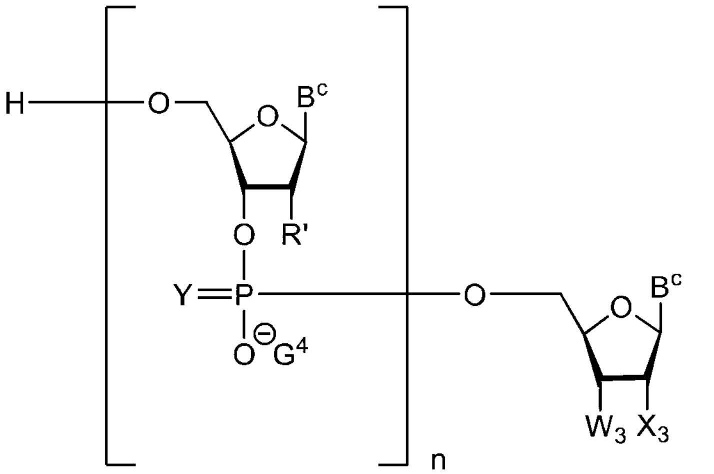

( 2' )

(wherein

Y and n are as described in claim 12,
$B^c$, each independently identical to or different from each other, represents a nucleobase,
$G^4$, each independently identical to or different from each other, represents a hydrogen ion, an alkali metal ion, an

ammonium ion, an alkyl ammonium ion, or a hydroxy alkyl ammonium ion,
R', each independently identical to or different from each other, represents a hydroxy group, a hydrogen atom, a fluorine atom, a methoxy group, a 2-methoxyethyl group, or an OQ' group,
Q' is as described in claim 12, and
$X_3$ and $W_3$ each independently represent a hydroxy group, or
$X_3$ represents an R' group and $W_3$ represents a hydroxy group, and
as defined in formula (1), a non-nucleotide linker may be incorporated between nucleotides)

, which comprises the step described in claim 12 and further a step of removing a group represented by Z from the oligonucleotide represented by formula (2) which is produced by the step, and a step of removing protecting groups for hydroxy groups and nucleobases.

**16.** The production method according to any one of claims 12 to 15, wherein the oligonucleotide is an oligonucleotide containing ribonucleoic acid (RNA).

**17.** The production method according to claim 12 wherein the oligonucleotide is an oligonucleotide containing ribonucleic acid (RNA), and a protecting group for the hydroxy group at the 2' position of the ribose is a protecting group represented by formula (6):

formula (6):

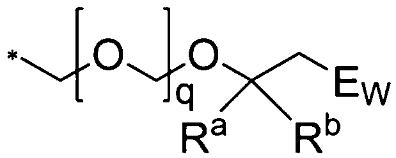

(6)

(wherein

q represents any integer of 0 to 5,
$R^a$ and $R^b$, each independently identical to or different from each other, represent a methyl group, an ethyl group or a hydrogen atom,
mark * represents the bonding point with the oxygen atom derived from the hydroxy group at the 2' position of the ribose, and
$E_W$ represents an electron-withdrawing group).

**18.** The production method according to claim 17 wherein q is 0 or 1, $R^a$ and $R^b$, each independently identical to or different from each other, represent a methyl group or a hydrogen atom, and $E_W$ is a cyano group.

**19.** The production method according to any one of claims 12 to 18 wherein n is any integer of 1 to 200.

**20.** The production method according to any one of claims 1 to 19 wherein the obtained oligonucleotide is an oligonucleotide of 100 mer or more.

**21.** The production method according to any one of claims 1 to 20 wherein the molar ratio of the used amounts of the thiol to the oligonucleotide wherein the hydroxy group at the 5' terminal is protected with the protecting group that can be removed under acidic conditions is 1 or more.

**22.** The production method according to any one of claims 1 to 21 wherein the molar ratio of the used amounts of the thiol to the acid is 1 to 100.

**Patentansprüche**

**1.** Ein Verfahren zur Herstellung eines Oligonukleotids durch ein Festphasensyntheseverfahren, welches Umsetzen eines Oligonukleotids, wobei die Hydroxygruppe an dem 5'-Terminus mit einer Schutzgruppe geschützt ist, die unter sauren Bedingungen entfernt werden kann, umfasst, **dadurch gekennzeichnet, dass** das Umsetzen mit einer Säure

in Gegenwart eines Thiols durchgeführt wird, um die Schutzgruppe für die Hydroxygruppe an dem 5'-Terminus zu entfernen.

2. Das Herstellungsverfahren nach Anspruch 1, wobei das Thiol ein C2-C20-Alkylthiol oder ein C4-C8-Cycloalkylthiol ist.

3. Das Herstellungsverfahren nach Anspruch 1 oder 2, wobei das Thiol 1-Dodecanthiol oder Cyclohexanthiol ist.

4. Das Herstellungsverfahren nach einem der Ansprüche 1 bis 3, wobei die Schutzgruppe für die Hydroxygruppe an dem 5'-Terminus eine Schutzgruppe, dargestellt durch die folgende Formel, ist:

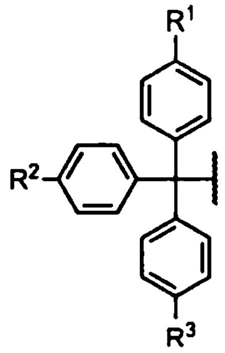

(wobei $R^1$, $R^2$ und $R^3$, jeweils unabhängig gleich oder voneinander verschieden, ein Wasserstoffatom oder eine Alkoxygruppe darstellen).

5. Das Herstellungsverfahren nach einem der Ansprüche 1 bis 4, wobei die Schutzgruppe für die Hydroxygruppe an dem 5'-Terminus eine 4,4'-Dimethoxytritylgruppe (DMTr-Gruppe) ist.

6. Das Herstellungsverfahren nach einem der Ansprüche 1 bis 5, wobei die Säure Trifluoressigsäure, Dichloressigsäure, Trifluormethansulfonsäure, Trichloressigsäure, Methansulfonsäure, Salzsäure, Essigsäure oder p-Toluolsulfonsäure ist.

7. Das Herstellungsverfahren nach einem der Ansprüche 1 bis 5, wobei die Säure Dichloressigsäure ist.

8. Das Herstellungsverfahren nach Anspruch 7, wobei die Säure Dichloressigsäure bei gleichzeitiger Anwesenheit eines aprotischen inerten Lösungsmittels mit einem niedrigeren Siedepunkt als Dichloressigsäure ist.

9. Das Herstellungsverfahren nach Anspruch 8, wobei das aprotische inerte Lösungsmittel mit einem niedrigeren Siedepunkt als Dichloressigsäure ein oder mehrere Lösungsmittel, ausgewählt aus Dichlormethan, Acetonitril oder einem aromatischen organischen Lösungsmittel, ist.

10. Das Herstellungsverfahren nach Anspruch 9, wobei das aromatische organische Lösungsmittel Toluol ist.

11. Das Herstellungsverfahren nach Anspruch 7, wobei die Dichloressigsäure Dichloressigsäure ist, wobei das Molverhältnis von Formaldehyd zu Dichloressigsäure 81 $\times$ $10^{-5}$ oder weniger beträgt und das Molverhältnis von Dichloressigsäureanhydrid zu Dichloressigsäure 20 $\times$ $10^{-5}$ oder weniger beträgt.

12. Das Herstellungsverfahren nach einem der Ansprüche 1 bis 11, wobei das Oligonukleotid, wobei die Hydroxygruppe an dem 5'-Terminus mit der Schutzgruppe geschützt ist, die unter sauren Bedingungen entfernt werden kann, ein Oligonukleotid, dargestellt durch Formel (1), ist:

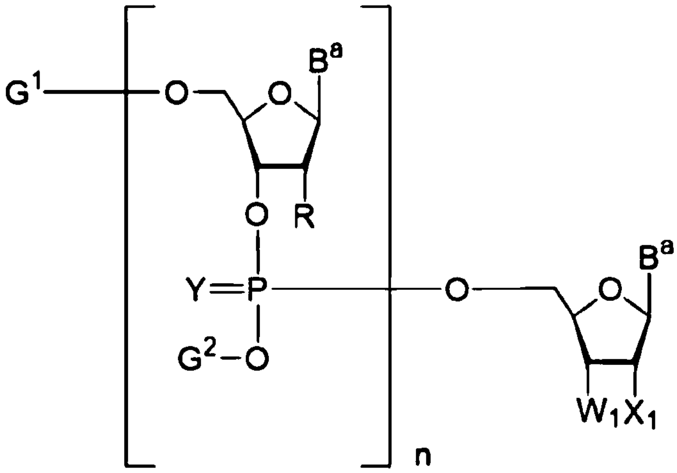

(1)

(wobei

$G^1$ eine Schutzgruppe für eine Hydroxygruppe darstellt,
$G^2$ eine Schutzgruppe für eine Hydroxygruppe darstellt,
$B^a$, jeweils unabhängig gleich oder voneinander verschieden, eine Nukleobase darstellt, die mit einer Schutzgruppe geschützt sein kann,
R, jeweils unabhängig gleich oder voneinander verschieden, eine geschützte Hydroxygruppe, ein Wasserstoffatom, ein Fluoratom, eine Methoxygruppe, eine 2-Methoxyethylgruppe oder eine OQ'-Gruppe darstellt,
Q', jeweils unabhängig gleich oder voneinander verschieden, eine Methylengruppe, die an das Kohlenstoffatom an der 4'-Position der Ribose gebunden ist, eine Ethylengruppe, die an das Kohlenstoffatom an der 4'-Position der Ribose gebunden ist, oder eine Ethylidengruppe, die an das Kohlenstoffatom an der 4'-Position der Ribose gebunden ist, darstellt,
Y, jeweils unabhängig gleich oder voneinander verschieden, ein Sauerstoffatom oder ein Schwefelatom darstellt,
n eine ganze Zahl von 1 bis 300 darstellt,
$W_1$ eine OZ-Gruppe darstellt und $X_1$ eine R-Gruppe darstellt, oder
$W_1$ eine OV-Gruppe darstellt und $X_1$ eine OZ-Gruppe darstellt,
V eine Schutzgruppe für eine Hydroxygruppe darstellt,
Z eine Gruppe mit einer Struktur ist, die aus einem festen Träger und einer Verbindungsgruppe besteht, und wenn n eine ganze Zahl von 2 oder mehr ist, ein Nicht-Nukleotid-Linker zwischen den jeweiligen Nukleotiden in dem durch Formel (1) dargestellten Nukleinsäuremolekül eingebaut sein kann), und
das Nukleotid, wobei die Schutzgruppe für die Hydroxygruppe an dem 5'-Terminus entfernt ist, ein Oligonukleotid, dargestellt durch Formel (2), ist:

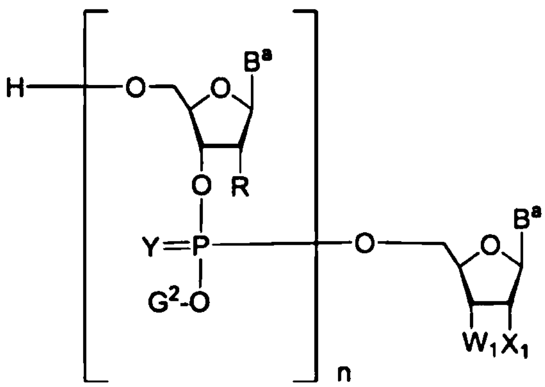

(2)

(wobei

$G^2$, $B^a$, R, Y, $X_1$, $W_1$ und n wie vorstehend beschrieben sind und
wie in Formel (1) definiert, ein Nicht-Nukleotid-Linker zwischen Nukleotiden eingebaut sein kann).

**13.** Das Herstellungsverfahren nach Anspruch 12, wobei das Oligonukleotid, wobei die Hydroxygruppe an dem 5'-Terminus mit der Schutzgruppe geschützt ist, die unter sauren Bedingungen entfernt werden kann, ein Oligonukleotid, dargestellt durch Formel (1'), ist:

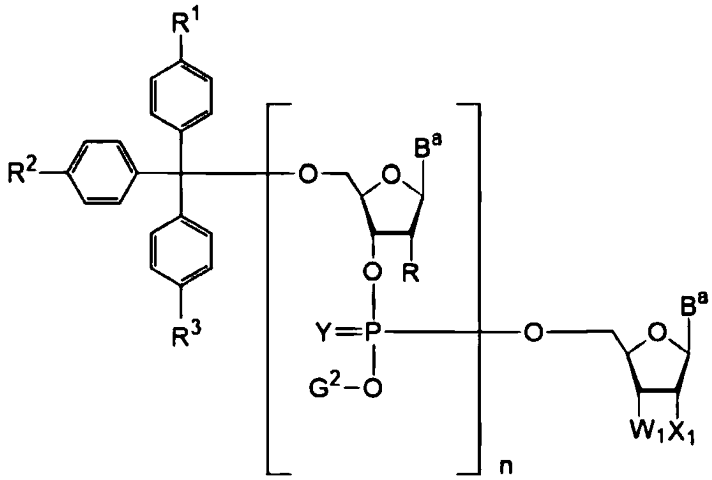

(1')

(wobei

$G^2$, $B^a$, R, Y, $X_1$, $W_1$ und n wie in Anspruch 12 beschrieben sind und
$R^1$, $R^2$ und $R^3$, jeweils unabhängig gleich oder voneinander verschieden, ein Wasserstoffatom oder eine Alkoxygruppe darstellen).

**14.** Das Herstellungsverfahren nach Anspruch 13, wobei $R^1$ und $R^2$ Methoxygruppen sind und $R^3$ ein Wasserstoffatom ist.

**15.** Ein Herstellungsverfahren für ein Oligonukleotid, dargestellt durch Formel (2'):

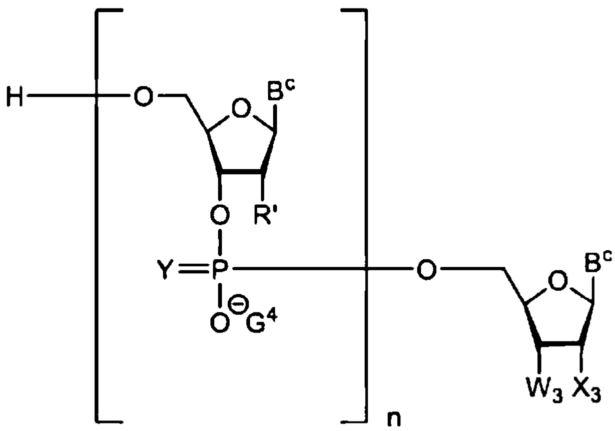

( 2' )

(wobei

Y und n wie in Anspruch 12 beschrieben sind,
$B^c$, jeweils unabhängig gleich oder voneinander verschieden, eine Nukleobase darstellt, $G^4$, jeweils unabhängig gleich oder voneinander verschieden, ein Wasserstoffion, ein Alkalimetallion, ein Ammoniumion, ein Alkylammoniumion oder ein Hydroxyalkylammoniumion darstellt,
R', jeweils unabhängig gleich oder voneinander verschieden, eine Hydroxygruppe, ein Wasserstoffatom, ein Fluoratom, eine Methoxygruppe, eine 2-Methoxyethylgruppe oder eine OQ'-Gruppe darstellt,
Q' wie in Anspruch 12 beschrieben ist, und
$X_3$ und $W_3$ jeweils unabhängig eine Hydroxygruppe darstellen oder
$X_3$ eine R'-Gruppe darstellt und $W_3$ eine Hydroxygruppe darstellt und

wie in Formel (1) definiert, ein Nicht-Nukleotid-Linker zwischen Nukleotiden eingebaut sein kann), das den in Anspruch 12 beschriebenen Schritt und ferner einen Schritt des Entfernens einer Gruppe, dargestellt durch Z, aus dem Oligonukleotid, dargestellt durch Formel (2), das durch den Schritt hergestellt wird, und einen Schritt des Entfernens von Schutzgruppen für Hydroxygruppen und Nukleobasen umfasst.

**16.** Das Herstellungsverfahren nach einem der Ansprüche 12 bis 15, wobei das Oligonukleotid ein Ribonukleinsäure (RNA) enthaltendes Oligonukleotid ist.

**17.** Das Herstellungsverfahren nach Anspruch 12, wobei das Oligonukleotid ein Ribonukleinsäure (RNA) enthaltendes Oligonukleotid ist und eine Schutzgruppe für die Hydroxygruppe an der 2'-Position der Ribose eine Schutzgruppe, dargestellt durch Formel (6), ist:

Formel (6):

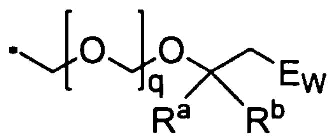

(6)

(wobei

q eine ganze Zahl von 0 bis 5 darstellt,
$R^a$ und $R^b$, jeweils unabhängig gleich oder voneinander verschieden, eine Methylgruppe, eine Ethylgruppe oder ein Wasserstoffatom darstellen,
Zeichen * den Bindungspunkt mit dem Sauerstoffatom darstellt, das von der Hydroxygruppe an der 2'-Position der Ribose abgeleitet ist, und
$E_W$ eine elektronenziehende Gruppe darstellt).

**18.** Das Herstellungsverfahren nach Anspruch 17, wobei q 0 oder 1 ist, $R^a$ und $R^b$, jeweils unabhängig gleich oder voneinander verschieden, eine Methylgruppe oder ein Wasserstoffatom darstellen und $E_W$ eine Cyanogruppe ist.

**19.** Das Herstellungsverfahren nach einem der Ansprüche 12 bis 18, wobei n eine ganze Zahl von 1 bis 200 ist.

**20.** Das Herstellungsverfahren nach einem der Ansprüche 1 bis 19, wobei das erhaltene Oligonukleotid ein 100 mer Oligonukleotid oder mehr ist.

**21.** Das Herstellungsverfahren nach einem der Ansprüche 1 bis 20, wobei das Molverhältnis der verwendeten Mengen des Thiols zu dem Oligonukleotid, wobei die Hydroxygruppe an dem 5'-Terminus mit der Schutzgruppe geschützt ist, die unter sauren Bedingungen entfernt werden kann, 1 oder mehr beträgt.

**22.** Das Herstellungsverfahren nach einem der Ansprüche 1 bis 21, wobei das Molverhältnis der verwendeten Mengen des Thiols zu der Säure 1 bis 100 beträgt.

## Revendications

**1.** Méthode de production d'un oligonucléotide par une méthode de synthèse en phase solide, qui comprend la réaction d'un oligonucléotide dans lequel le groupe hydroxy à l'extrémité 5' est protégé par un groupe protecteur qui peut être éliminé dans des conditions acides, **caractérisée en ce que** la réaction est mise en œuvre avec un acide en présence d'un thiol afin que le groupe protecteur pour le groupe hydroxy à l'extrémité 5' soit éliminé.

**2.** Méthode de production selon la revendication 1, dans laquelle le thiol est un alkylthiol en $C_2$ à $C_{20}$, ou un cycloalkylthiol en $C_4$ à $C_8$.

**3.** Méthode de production selon la revendication 1 ou 2, dans laquelle le thiol est le 1-dodécanethiol ou le cyclohexanethiol.

4. Méthode de production selon l'une quelconque des revendications 1 à 3, dans laquelle le groupe protecteur pour le groupe hydroxy à l'extrémité 5' est un groupe protecteur représenté par la formule suivante :

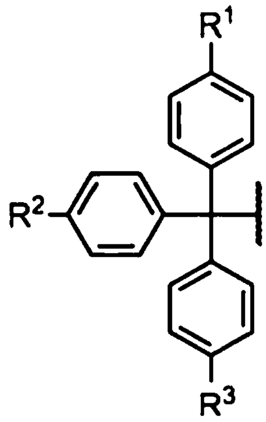

(dans laquelle$R^1$, $R^2$ et $R^3$, chacun indépendamment identiques ou différents les uns des autres, représentent un atome d'hydrogène ou un groupe alcoxy).

5. Méthode de production selon l'une quelconque des revendications 1 à 4, dans laquelle le groupe protecteur pour le groupe hydroxy à l'extrémité 5' est un groupe 4,4'-diméthoxytrityle (groupe DMTr).

6. Méthode de production selon l'une quelconque des revendications 1 à 5, dans laquelle l'acide est l'acide trifluoroacétique, l'acide dichloroacétique, l'acide trifluorométhanesulfonique, l'acide trichloroacétique, l'acide méthanesulfonique, l'acide chlorhydrique, l'acide acétique, ou l'acide p-toluènesulfonique.

7. Méthode de production selon l'une quelconque des revendications 1 à 5, dans laquelle l'acide est l'acide dichloroacétique.

8. Méthode de production selon la revendication 7, dans laquelle l'acide est l'acide dichloroacétique qui coexiste avec un solvant aprotique inerte ayant un point d'ébullition inférieur à l'acide dichloroacétique.

9. Méthode de production selon la revendication 8, dans laquelle le solvant aprotique inerte ayant un point d'ébullition inférieur à l'acide dichloroacétique est un ou plusieurs solvants choisis parmi le dichlorométhane, l'acétonitrile, et un solvant organique aromatique.

10. Méthode de production selon la revendication 9, dans laquelle le solvant organique aromatique est le toluène.

11. Méthode de production selon la revendication 7, dans laquelle l'acide dichloroacétique est l'acide dichloroacétique dans lequel le rapport molaire du formaldéhyde à l'acide dichloroacétique est de $81 \times 10^{-5}$ ou moins, et le rapport molaire de l'anhydride dichloroacétique à l'acide dichloroacétique est de $20 \times 10^{-5}$ ou moins.

12. Méthode de production selon l'une quelconque des revendications 1 à 11, dans laquelle l'oligonucléotide dans lequel le groupe hydroxy à l'extrémité 5' est protégé par le groupe protecteur qui peut être éliminé dans des conditions acides est un oligonucléotide représenté par la formule (1) :

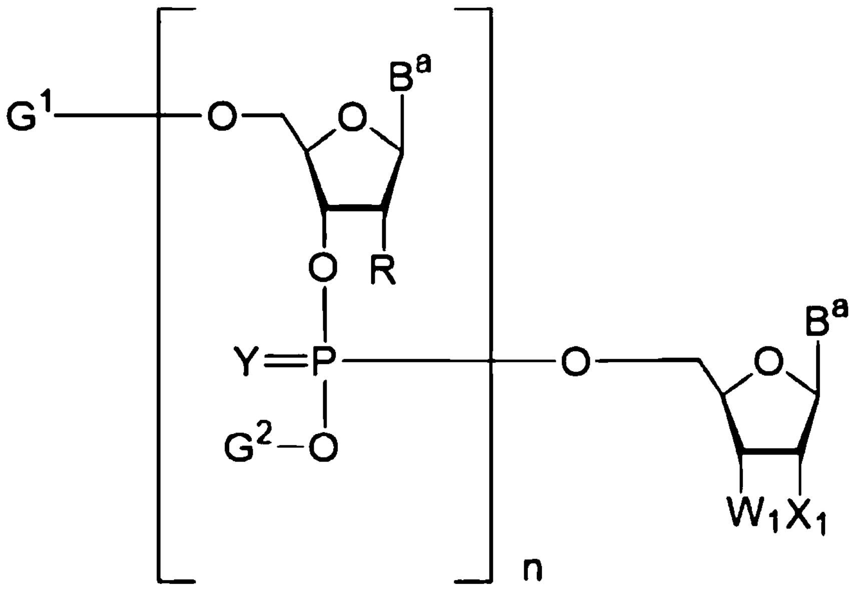

(1)

(dans laquelle

$G^1$ représente un groupe protecteur pour un groupe hydroxy,
$G^2$ représente un groupe protecteur pour un groupe hydroxy,
$B^a$ représente une nucléobase, chacune indépendamment identique ou différente les unes des autres, qui peut être protégée par un groupe protecteur,
R, chacun indépendamment identique ou différent les uns des autres, représente un groupe hydroxy protégé, un atome d'hydrogène, un atome de fluor, un groupe méthoxy, un groupe 2-méthoxyéthyle ou un groupe OQ',
Q', chacun indépendamment identique ou différent les uns des autres, représente un groupe méthylène qui est lié à l'atome de carbone à la position 4' du ribose, un groupe éthylène qui est lié à l'atome de carbone à la position 4' du ribose, ou un groupe éthylidène qui est lié à l'atome de carbone à la position 4' du ribose,
Y, chacun indépendamment identique ou différent les uns des autres, représente un atome d'oxygène ou un atome de soufre,
n représente un entier quelconque de 1 à 300,
$W_1$ représente un groupe OZ et $X_1$ représente un groupe R, ou
$W_1$ représente un groupe OV et $X_1$ représente un groupe OZ,
V représente un groupe protecteur pour un groupe hydroxy,
Z est un groupe ayant une structure consistant en un support solide et un groupe de liaison, et
quand n est un entier valant 2 ou plus, un connecteur non nucléotidique peut être incorporé entre des nucléotides respectifs dans la molécule d'acide nucléique représentée par la formule (1)),
et le nucléotide dans lequel le groupe protecteur pour le groupe hydroxy à l'extrémité 5' est éliminé est un oligonucléotide représenté par la formule (2) :

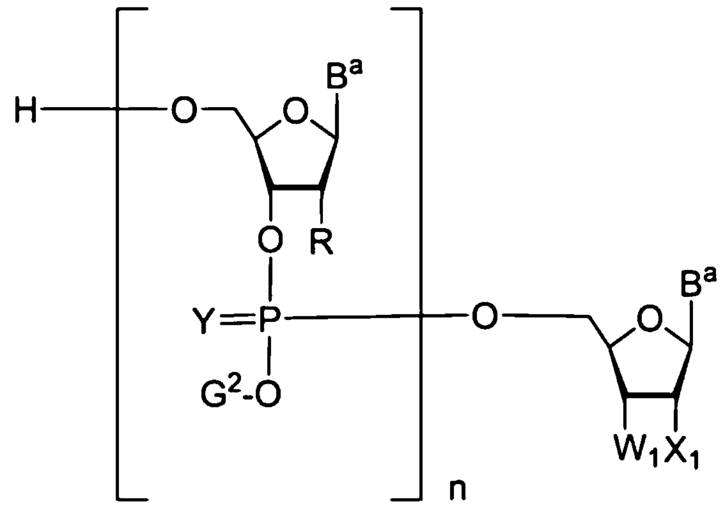

(2)

(dans laquelle

$G^2$, $B^a$, R, Y, $X_1$, $W_1$ et n sont tels que décrits ci-dessus, et
comme défini dans la formule (1), un connecteur non nucléotidique peut être incorporé entre des nucléotides).

**13.** Méthode de production selon la revendication 12, dans laquelle l'oligonucléotide dans lequel le groupe hydroxy à l'extrémité 5' est protégé par le groupe protecteur qui peut être éliminé dans des conditions acides est un oligonucléotide représenté par la formule (1') :

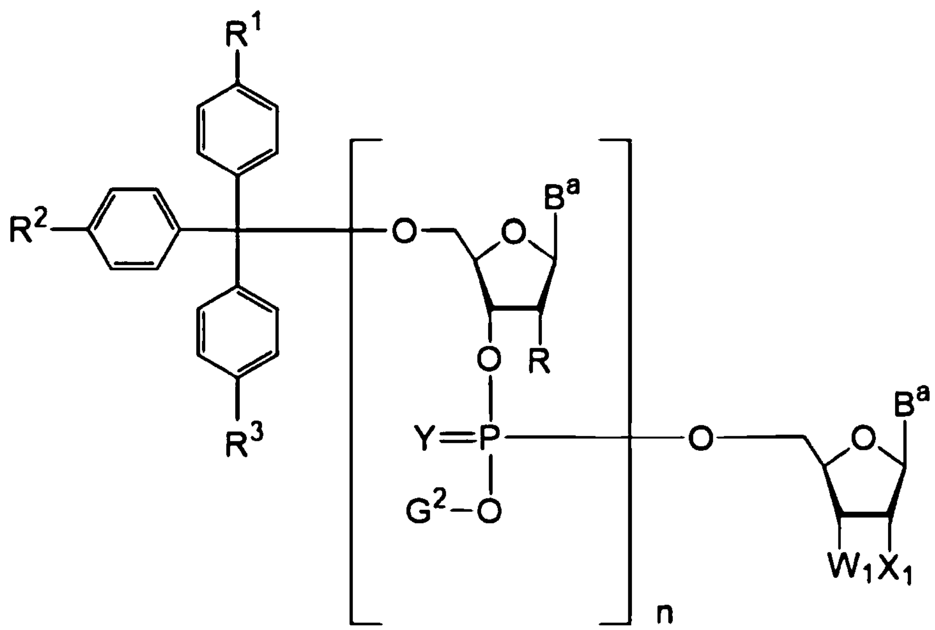

(1')

(dans laquelle

$G^2$, $B^a$, R, Y, $X_1$, $W_1$ et n sont tels que décrits dans la revendication 12, et
$R^1$, $R^2$ et $R^3$, chacun indépendamment identiques ou différents les uns des autres, représentent un atome d'hydrogène ou un groupe alcoxy).

**14.** Méthode de production selon la revendication 13, dans laquelle $R^1$ et $R^2$ sont des groupes méthoxy, et $R^3$ est un atome d'hydrogène.

**15.** Méthode de production d'un oligonucléotide représenté par la formule (2') :

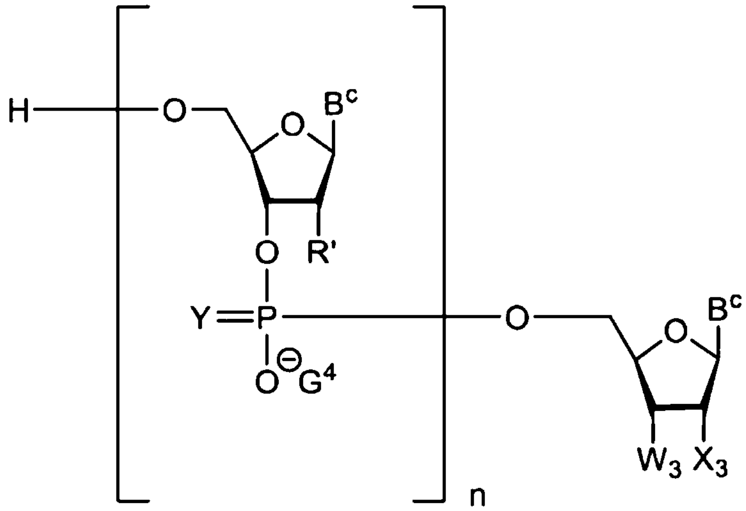

( 2' )

(dans laquelle

Y et n sont tels que décrits dans la revendication 12,
$B^c$, chacun indépendamment identique ou différent les uns des autres, représente une nucléobase,
$G^4$, chacun indépendamment identique ou différent les uns des autres, représente un ion hydrogène, un ion de métal alcalin, un ion ammonium, un ion alkylammonium, ou un ion hydroxyalkylammonium,
R', chacun indépendamment identique ou différent les uns des autres, représente un groupe hydroxy, un atome d'hydrogène, un atome de fluor, un groupe méthoxy, un groupe 2-méthoxyéthyle, ou un groupe OQ',
Q' est tel que décrit dans la revendication 12, et
$X_3$ et $W_3$ représentent chacun indépendamment un groupe hydroxy, ou
$X_3$ représente un groupe R' et $W_3$ représente un groupe hydroxy, et
comme défini dans la formule (1), un connecteur non nucléotidique peut être incorporé entre des nucléotides) qui comprend l'étape décrite dans la revendication 12 et en outre une étape d'élimination d'un groupe représenté par Z de l'oligonucléotide représenté par la formule (2) qui est produit par l'étape, et une étape d'élimination des groupes protecteurs pour les groupes hydroxy et les nucléobases.

**16.** Méthode de production selon l'une quelconque des revendications 12 à 15, dans laquelle l'oligonucléotide est un oligonucléotide contenant un acide ribonucléique (ARN).

**17.** Méthode de production selon la revendication 12, dans laquelle l'oligonucléotide est un oligonucléotide contenant un acide ribonucléique (ARN), et le groupe protecteur pour le groupe hydroxy à la position 2' du ribose est un groupe protecteur représenté par la formule (6) :

formule (6)

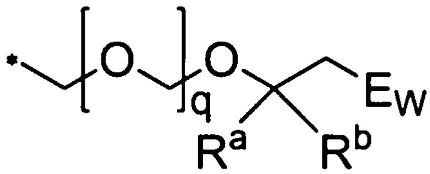

( 6 )

(dans laquelle

q représente un entier quelconque de 0 à 5,
$R^a$ et $R^b$, chacun identiques ou différents les uns des autres, représentent un groupe méthyle, un groupe éthyle ou un atome d'hydrogène,
le caractère * représente le point de liaison avec l'atome d'oxygène dérivé du groupe hydroxy à la position 2' du ribose, et
$E_W$ représente un groupe électro-attracteur).

**18.** Méthode de production selon la revendication 17, dans laquelle q vaut 0 ou 1, $R^a$ et $R^b$, chacun indépendamment identiques ou différents les uns des autres, représentent un groupe méthyle ou un atome d'hydrogène, et $E_W$ est un groupe cyano.

**19.** Méthode de production selon l'une quelconque des revendications 12 à 18, dans laquelle n est un entier quelconque de 1 à 200.

**20.** Méthode de production selon l'une quelconque des revendications 1 à 19, dans laquelle l'oligonucléotide obtenu est un oligonucléotide 100-mer ou plus.

**21.** Méthode de production selon l'une quelconque des revendications 1 à 20, dans laquelle le rapport molaire des quantités utilisées du thiol à l'oligonucléotide dans lequel le groupe hydroxy à l'extrémité 5' est protégé par le groupe protecteur qui peut être éliminé dans des conditions acides est de 1 ou plus.

**22.** Méthode de production selon l'une quelconque des revendications 1 à 21, dans laquelle le rapport molaire des quantités utilisées du thiol à l'acide est de 1 à 100.

[Fig. 1]

Scheme A

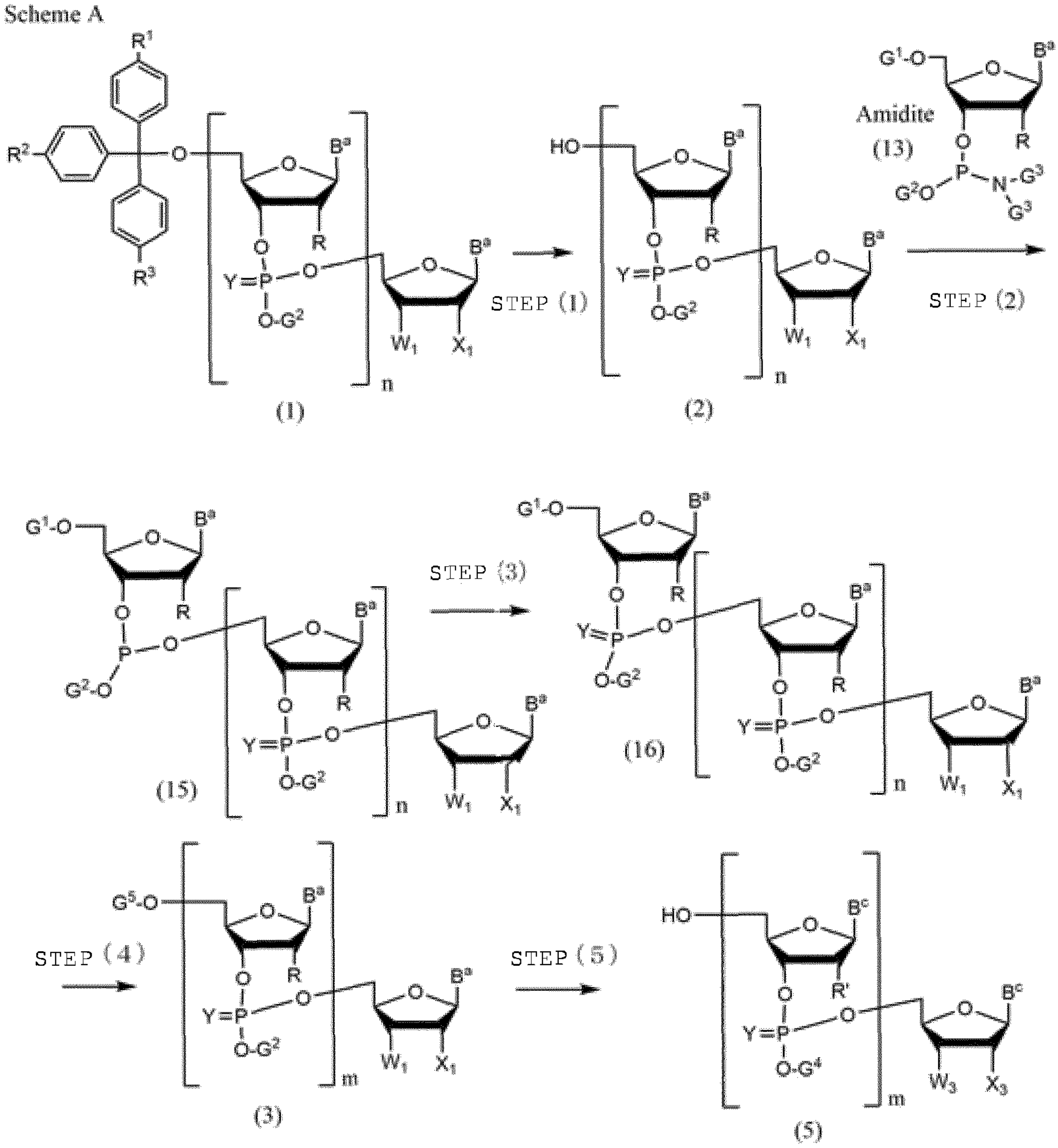

(1)
(2)
(15)
(16)
(3)
(5)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022172158 A
- WO 2012157723 A1
- US 5510476 B1
- WO 2020227618 A2
- WO 2022103842 A1
- US 10072261 B1
- WO 2019208571 A1
- JP 2005520547 A
- WO 2022172994 A1
- JP 4705716 B
- WO 2006022323 A
- WO 2013027843 A
- WO 2019208571 A
- WO 2013027843 A1
- WO 2006022323 A1
- WO 2019074110 A1
- WO 2012005368 A1
- WO 2018182008 A1
- JP 3745226 B
- WO 2001053528 A1
- JP 2014221817 A
- JP 5157168 B
- JP 5554881 B
- WO 2019060442 A1
- JP 2015523856 A
- JP 2017537626 A


### Non-patent literature cited in the description


- **J. AM.** *Chem. Society*, 2020, vol. 142, 16610
- **ZHOU XUAN et al.** *The Journal of Organic Chemistry*, 2021, vol. 87 (4), 2087-2110
- **MITSUAKI SEKIGUCHI**. *Pharmaceutical and Medical Device Regulatory Science*, 2020, vol. 51 (1), 11-21
- **N. M. EL ZAHAR et al.** *Biomedical Chromatography*, 2017, vol. 32 (1), e4088
- **XIULONG, SHEN et al.** *Nucleic Acids Research*, 2018, vol. 46 (46), 1584-1600
- **DANIEL O'REILLY et al.** *Nucleic Acids Research*, 2019, vol. 47 (2), 546-558
- **O'REILLY et al.** *Nucleic Acids Research*, 2019, vol. 47 (2), 546-558
- *Nucleic Acids Research*, 2019, vol. 47 (2), 547